# EUROPÄISCHE PATENTSCHRIFT

(11) **EP 1 745 133 B1**
(45) Veröffentlichungstag und Bekanntmachung des Hinweises auf die Patenterteilung: **15.07.2009**
(21) Anmeldenummer: 05745555.2
(22) Anmeldetag: 04.05.2005
(51) Int. Cl.: C12P 7/02, C12P 17/00

(54) **VERFAHREN ZUR HERSTELLUNG OPTISCH AKTIVER ALKOHOLE AUS ALKANONEN UNTER VERWENDUNG EINER DEHYDROGENASE AUS AZOARCUS**
PROCESS FOR THE PREPARATION OF OPTICALLY ACTIVE ALCOHOLS EMPLOYING A DEHYDROGENASE DERIVED FROM AZOARCUS
PROCÉDÉ DE PRÉPARATION D'ALCOOLS OPTIQUEMENT ACTIFS EN UTILISANT UNE DESHYDROGENASE DÉRIVÉE DE AZOARCUS

(30) Priorität: 05.05.2004 DE 102004022686
(43) Veröffentlichungstag der Anmeldung: 24.01.2007
(73) Patentinhaber: BASF SE, 67056 Ludwigshafen (DE)
(72) Erfinder: STÜRMER, Rainer, 67127 Rödersheim-Gronau (DE); KESSELER, Maria, 68167 Mannheim (DE); HAUER, Bernhard, 67136 Fussgönheim (DE); FRIEDRICH, Thomas, 64283 Darmstadt (DE); BREUER, Michael, 67117 Limburgerhof (DE); SCHRÖDER, Hartwig, 69226 Nussloch (DE)
(86) Internationale Anmeldenummer: PCT/EP2005/004872
(87) Internationale Veröffentlichungsnummer: WO 2005/108590

(56) Entgegenhaltungen:
- EP-A- 1 405 917
- WO-A-20/04090094
- WO-A-20/05033094
- ITOH, N. ET AL.: "Chiral alcohol production by NADH-dependent phenylacetaldehyde reductase coupled with in situ regeneration of NADH" EUROPEAN JOURNAL OF BIOCHEMISTRY, Bd. 269, Nr. 9, Mai 2002 (2002-05), Seiten 2394-2402, XP002221911
- HAMADA, H. ET AL.: "Asymmetric synthesis of (R)-2-chloro-1-(m-chlorophenyl)ethanol using acetone powder of Geotrichum candidum" BIOTECHNOLOGY LETTERS, Bd. 23, Nr. 19, Oktober 2001 (2001-10), Seiten 1603-1606, XP008033821
- NAKAMURA, K. ET AL.: "Asymmetric Reduction of Trifluoromethyl Ketones Containing a Sulfur Functionality by the Alcohol Dehydrogenase from Geotrichum" TETRAHEDRON, Bd. 54, Nr. 29, 16. Juli 1998 (1998-07-16), Seiten 8393-8402, XP004124021
- STILLGER, T. ET AL.: "Überwindung von thermodynamischen Limitierungen in substratgekoppelten Cofaktorregenerierungsverfahren" CHEMIE INGENIEUR TECHNIK, Bd. 74, 2002, Seiten 1035-1039, XP002358593 in der Anmeldung erwähnt
- RABUS, R. ET AL.: "Genes involved in the anaerobic degradation of ethylbenzene in a denitrifying bacterium, strain EbN1" ARCHIVES OF MICROBIOLOGY, Bd. 178, Nr. 6, Dezember 2002 (2002-12), Seiten 506-516, XP002358594
- KNIEMEYER, O. & HEIDER, J.: "(S)-1-Phenylethanol dehydrogenase of Azoarcus sp. strain EbN1, an enzyme of anaerobic ethylbenzene catabolism" ARCHIVES OF MICROBIOLOGY, Bd. 176, Nr. 1-2, Juli 2001 (2001-07), Seiten 129-135, XP002358595

## Beschreibung

Die vorliegende Erfindung betrifft ein Verfahren zur Herstellung von optisch aktiven Alkanolen durch enzymatische Reduktion der entsprechenden Ketone, insbesondere die Herstellung von (1S)-3-Methylamino-1-(2-thienyl)-propan-1-ol und (1S)-3-Chlor-1-(2-thienyl)-propan-1-ol.

### Stand der Technik:

(1S)-3-Methylamino-1-(2-thienyl)-propan-1-ol ("Duloxetinealkohol") ist Baustein in der Duloxetine-Synthese. Duloxetine® ist ein Pharmawirkstoff, der sich momentan in der Zulassung befindet und im Indikationsgebiet Depression und Inkontinenz eingesetzt werden soll.

EP-B-0273658 beschreibt ein Verfahren zur Herstellung der korrespondierenden Base von Duloxetine durch Umsetzung von 2-Acetylthiophen in einer Mannich-Reaktion mit Formaldehyd und Dimethylamin, Reduktion der Ketogruppe der dabei erhaltenen Mannich-Base zum racemischen (S)-3-N,N-Dimethylamino-1-(thien-2-yl)propan-1-ol, Veretherung der Alkoholfunktion mit Naphthylfluorid und schließlich Umwandlung der Dimethylamino-Gruppe in eine Methylamino-Funktion. Das gewünschte Enantiomer des Naphtylethers erhält man durch Einsatz chiraler Ausgangsmaterialien oder durch Racemattrennung auf der Stufe des Endprodukts, beispielsweise über die Salze mit optisch aktiven Säuren oder Chromatographie an einer chiralen stationären Phase.

US-5,362,886 beschreibt ein analoges Verfahren, bei dem das nach Reduktion der Ketogruppe erhaltene racemische Propanol mit S-Mandelsäure versetzt wird. Das hierbei erhaltene S-Enantiomer des Alkohols wird in die nachfolgenden Reaktionsstufen eingesetzt.

EP-A-0457559 beschreibt ebenfalls ein der EP-B-0273658 analoges Verfahren. Hierbei wird die Ketogruppe der Mannich-Base mit dem asymmetrischen Reduktionssystem LAH-lcb (Lithiumaluminiumhydrid-[(2R,2S)-(-)-4-Dimethylamino-1,2-diphenyl-3-methyl-2-butanol]) zum Alkohol in Form des S-Enantiomers reduziert. Nachteilig hierbei ist neben den Kosten die Empfindlichkeit des Reduktionssystems LAH-lcb, das nur wenige Minuten stabil ist.

W. J. Wheeler und F. Kuo beschreiben in Journal of Labelled Compounds and Radiopharmaceuticals, Band XXXVI, Nr. 3, Seite 213 bis 223 ein Verfahren zur Herstellung von Duloxetine. Hierzu wird Thiophen-2-carbonsäurechlorid in einer Stille-Kopplung mit Vinyl-tri-n-butylstannan in Gegenwart katalytischer Mengen Benzylchlorbis(triphenylphosphin)palladium(II) in DMPU (Dimethylpropylenharnstoff) zu 1-(Thien-2-yl)-propenon der Formel (V) 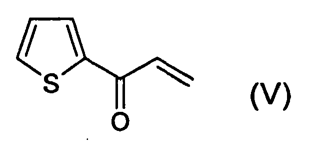 umgesetzt, welches anschließend durch Behandlung mit Chlorwasserstoff in 3-Chlor-1-(thien-2-yl)-propan-1-on der Formel (VI) 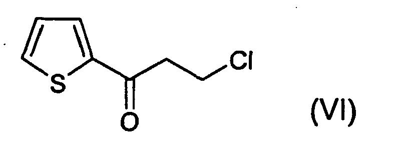 überführt wird. Das so erhaltene Chlorpropanon wird anschließend unter Verwendung eines chiralen Oxazaborylidins und BH₃ zu (S)-3-Chlor-1-(thien-2-yl)-propan-1-ol der Formel (VII) 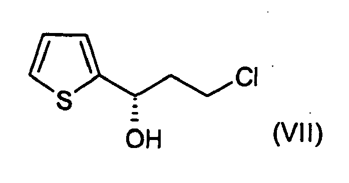 reduziert. Der so erhaltene Alkohol wird durch sukzessive Umsetzung mit Natriumiodid und anschließend mit Methylamin in (*S*)-3-Methylamino-1-(thien-2-yl)-propan-1-ol überführt. Durch nachfolgende sukzessive Umsetzung mit Natriumhydrid, 1-Fluomaphthalin und Chlorwasserstoff erhält man Duloxetine in Form des Hydrochlorids. 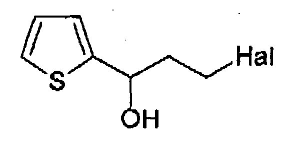 Hal = Halogen

T. Stillger et al. beschreiben in Chemie Ingenieur Technik (74) Seiten 1035-1039, 2002 substratgekoppelte Cofaktorregenerierungsverfahren zur enzymatischen enantioselektiven Reduktion von 5-Oxohexansäureethylester zu (S)-5-Hydroxyhexansäureethylester.

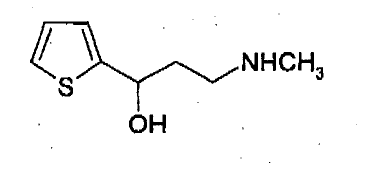

Itoh et al. , European Journal of Biochemistry, Bd. 269, Nr. 9, Mai 2002, Seiten 2394-2402 offenbart ein Verfahren zur Herstellung von (R)-2-Chlor-1-(m-chlorphenyl)ethanol aus m-Chlorphenacylchlorid unter Verwendung der Phenylacetaldehyd Reduktase aus Corynebacterium sp ST-10, welches rekombinant in E. coli exprimiert wird. Das verwendete Enzym wird jedoch leicht durch den Opferalkohol 2-Pentanol denaturiert.

WO 2005/033094 beschreibt ein Verfahren zur Herstellung von (S)-3-Chlor-1-(2-thienyl)-1- propanol aus 3-Chlor-1-(2-thieny)-1-propanon durch enzymatische enantioselektive Reduktion unter Verwendung von Alkoholdehydrogenasen aus Lactobacillus brevis und Candida magnoliae.NADH wird in Gegenwart sekundärer Alkohole regeneriert und (S)-3- Chlor-1-(2-thienyl)-1-Propanol wird zur Herstellung von Duloxetine verwendet.

### Kurze Beschreibung der Erfindung:

Der Erfindung lag daher die Aufgabe zugrunde, einen Weg zur stereospezifischen Reduktion von substituierten Alkanonen, wie dem 3-Methylamino-1-(2-thienyl)-propanon und dem 3-Chlor-1-(2-thienyl)-propanon, zu finden, wobei das Reaktionsverfahren auf kostengünstigem Weg möglichst quantitativ zum Produkt führen sollte.

Diese Aufgabe wurde durch den überraschenden Befund gelöst, dass Enzyme mit Dehydrogenase-Aktivität, die aus Mikroorganismen der Gattung *Azoarcus* herstellbar sind, zur stereospezifischen Katalyse der obigen Reaktion unter gleichzeitiger Cofaktorregenerierung befähigt sind.

Ein erster Gegenstand der Offenlegung betrifft ein Verfahren zur Herstellung von optisch aktiven-Alkanolen der Formel I 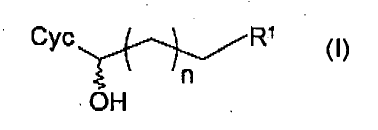 worin
- n: für einen ganzzahligen Wert von 0 bis 5 steht;
- Cyc: für einen gegebenenfalls substituierten, ein- oder mehrkernigen, gesättigten oder ungesättigten, carbocyclischen oder heterocyclischen Ring steht, und

- R¹: für Halogen, SH, OH, NO₂, NR²R³ oder NR²R³R⁴⁺X⁻ steht, wobei R², R³ und R⁴ unabhängig voneinander für H oder einen Niedrigalkyl- oder Niedrigalkoxy-Rest stehen und X⁻ für ein Gegenion steht,
wobei man in einem Alkanon der Formel II 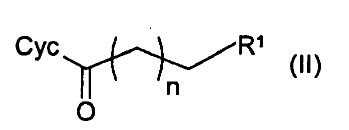 worin n, Cyc und R¹ die oben angegebenen Bedeutungen besitzen,
enthaltenden Medium, ein Enzym (E), ausgewählt aus den Klassen der Dehydrogenasen, Aldehydreduktasen und Carbonylreduktasen, inkubiert in Gegenwart von Reduktionsäquivalenten, wobei die Verbindung der Formel II zur Verbindung der Formel I enzymatisch reduziert wird, und die im Laufe der Reaktion verbrauchten Reduktionsäquivalente durch Umsetzen eines Opfer-Alkohols zum entsprechenden Opfer-Keton mit Hilfe des Enzyms (E) wieder regeneriert werden und das Opfer-Keton zumindest partiell aus dem Reaktionsmedium entfernt wird, und man das gebildete Produkt (I) isoliert

Geeignete Enzyme (E) sind insbesondere die Enzyme der Familien der Aldo-Keto-Reduktasen der Aldo-Keto-Reduktase-Superfamily (K.M.Bohren, B.Bullock, B.Wermuth und K.H.Gabbay J.Biol. Chem. 1989, 264, 9547-9551) und der kurzkettigen Alkoholdehydrogenasen / Reduktasen (shortchain alcohol dehydrogenases / reductases [SDR]) zählen. Die letztere Enzymgruppe ist ausführlich beschrieben zum Beispiel bei H.Jömvall, B.Persson, M.Krook, S.Atrian, R.Gonzalez-Duarte, J.Jeffery und D.Ghosh, Biochemistry, 1995, 34, S. 6003-6013 oder U.Oppermann, C.Filling, M.Hult, N.Shafqat, X.Q.Wu, M.Lindh, J.Shafqat, E.Nordling, Y.Kallberg, B.Persson und H.Jomvall, Chemico-Biological Interactions, 2003, 143, S. 247-253.
Innerhalb dieser genannten Enzymklassen sind die kurzkettigen Alkoholdehydrogenasen besonders gut geeignet.
Die Erfindung befrifft die Verwendung von Enzymen (E) im o.g. Verfahren, wobei E eine Polypeptidsequenz
(i) SEQ ID NO: 2 oder
(ii) bei der bis zu 25% der Aminosäurereste gegenüber SEQ ID NO:2 durch Deletion, Insertion, Substitution oder einer Kombination davon verändert sind und die noch mindestens 50% der enzymatischen Aktivität von SEQ ID NO:2 besitzt, besitzt.

In einer bevorzugten Ausführungsform dient das Verfahren zur Herstellung von Derivaten des 1-(2-thienyl)-(S)-propanol der Formel III 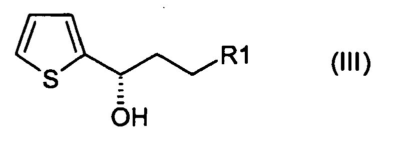 wobei R¹ = Cl oder NHCH₃ bedeuten,
wobei man in einem ein Derivat des 1-(2-thienyl)-propanons der Formel IV 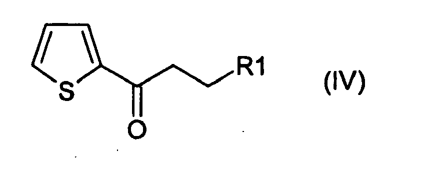 enthaltenden Medium diese Verbindung zur Verbindung der Formel III enzymatisch reduziert wird, und man das in im Wesentlichen enantiomerenreiner Form gebildete Produkt isoliert.

Ebenfalls offenbart ist die Verwendung eines Enzyms mit Dehydrogenase-Aktivität, das aus Mikroorganismen der Gattungen *Azoarcus, Azonexus, Azospira, A-zovibrio, Dechloromonas, Ferribacterium, Petrobacter, Propionivibrio, Quadricoccus, Rhodocyclus, Sterolibacterium, Thauera* und *Zoogloea* herstellbar ist.

Besonders bevorzugt werden Dehydrogenasen aus Arten der Gattung *Azoarcus.*

Aufgrund ihrer Aminosäuresequenz kann man die Phenylethanol-Dehydrogenase aus *Azoarcus* sp EbN1 zu den kurzkettigen Alkoholdehydrogenasen / Reduktasen (shortchain alcohol dehydrogenases / reductases [SDR]) zählen. Die Enzymgruppe ist ausführlich beschrieben zum Besipiel bei H.JÖrnvall, B.Persson, M.Krook, S.Atrian, R.Gonzalez-Duarte, J.Jeffery und D.Ghosh, Biochemistry, 1995, 34, S. 6003-6013 oder U.Oppermann, C.Filling, M.Hult, N.Shafqat, X.Q.Wu, M.Lindh, J.Shafqat, E.Nordling, Y.Kallberg, B.Persson und H.Jomvall, Chemico-Biological Interactions, 2003, 143, S. 247-253. Innerhalb der Gruppe der SDR können sich die Aminosäuresequenzen der einzelnen Vertreter stark voneinander unterscheiden. Dennoch ist bekannt, dass bestimmte Aminosäuren oder Aminosäurenbereiche innerhalb der Gruppe der SDR stark konserviert sind. In C.Filling, K.D.Bemdt, J.Benach, S.Knapp, T.Prozorovski, E.Nordling, R.Ladenstein, H.Jornvall und U.Oppermann, Journal of Biological Chemistry, 2002, 277, S. 25677-25684 sind wichtige konservierte Bereiche der SDR beschrieben.

Beispiele für Azoarcus-Arten sind *Azoarcus anaerobius, Azoarcus buckelii, Azoarcus communis, Azoarcus evansii, Azoarcus indigens, Azoarcus toluclasticus, Azoarcus tolulyticus, Azoarcus toluvorans, Azoarcus* sp., *Azoarcus* sp. 22Lin, *Azoarcus* sp. BH72, *Azoarcus* sp. CC-11, *Azoarcus* sp. CIB, *Azoarcus* sp. CR23, *Azoarcus* sp. EB1, *Azoarcus* sp. EbN1, *Azoarcus* sp. FL05, *Azoarcus* sp. HA, *Azoarcus* sp. HxN1, *Azoarcus* sp. mXyN1, *Azoarcus* sp. PbN1, *Azoarcus* sp. PH002, *Azoarcus* sp. T und *Azoarcus* sp. ToN1.

Besonders bevorzugt verwendet man Dehydrogenasen aus *Azoarcus* sp EbN1.

In einer Ausführungsform des Verfahrens ist das Enzym mit Dehydrogenase-Aktivität ausgewählt unter Enzymen, welche eine Aminosäuresequenz gemäß SEQ ID NO: 2 umfassen oder eine davon abgeleitete Sequenz, bei der bis zu 25%, % der Aminosäurereste durch eine Deletion, eine Substitution eine Insertion oder eine Kombination aus Deletion, Substitution und Insertion verändert worden sind, wobei die gegenüber SEQ ID NO: 2 veränderten Polypeptidsequenzen noch mindestens 50%, bevorzugt 65%, besonders bevorzugt 80% , insbesondere mehr als 90 % der enzymatischen Aktivität von SEQ ID NO:2 besitzten. In diesem Zusammenhang soll unter enzymatische Aktivität von SEQ ID NO:2 die Fähigkeit verstanden werden, die Ketone der Formel (IV) mit R¹ = Cl enantioselektiv zu dem (S)-Alkohol mit der allgemeinen Formel (III) zu reduzieren. Die genauen Bedingungen zur Ermittlung der enzymatischen Aktivität sind in Beispiel 3 und 4 wiedergegeben.

Das erfindungsgemäße Verfahren wird unter Zugabe von Reduktionsäquivalenten, insbesondere von NADH oder NADPH, durchgeführt. Zur Regenerierung der bei der Reaktion verbrauchten Reduktionsäquivalente wird ein Opfer-Alkohol, bevorzugt Isopropanol, 2-Butanol, 2-Pentanol, 2-Hexanol, 3-Hexanol eingesetzt, der unter den Reaktionsbedingungen durch das Enzym (E) zu dem entsprechenden Opfer-Keton oxidiert wird.

In einer bevorzugten Ausführungsform der Erfindung wird der zugesetzte Opfer-Alkohol nicht nur zur Regenerierung der verbrauchten Reduktionsäquivalente eingesetzt, sondern auch als Co-Lösungsmittel. Man arbeitet bevorzugt in einem flüssigen 2-Phasensystem, wobei die eine Phase aus Wasser oder mit Wasser mischbarem Lösungsmittel besteht, und die andere Phase aus dem Opfer-Alkohol besteht. Bevorzugt wird als Opfer-Alkohol 2-Pentanol eingesetzt.

Die Reduktionsäquivalente werden bevorzugt in einer Menge von 0,001 bis 100 mmol , besonders bevorzugt von 0,01 bis 1 mmol Reduktionsäquivalente pro Mol eingesetztem Alkanon (II) eingesetzt.

Bei dem erfindungsgemäßen Verfahren wird das bei der Regenerierung der verbrauchten Reduktionsäquivalente durch Oxidation des Opfer-Alkohols entstandene Opfer-Keton aus dem Reaktionsgemisch zumindest partiell entfernt. Bevorzugt entfernt man das gebildete Opfer-Keton vollständig aus dem Reaktionsmedium. Dies kann auf verschiedene Weise vorgenommen werden, beispielsweise durch selektive Membranen oder durch Extraktions- oder Destillationsverfahren. Bevorzugt wird die Destillation zur Entfernung des Ketons eingesetzt. Üblicherweise wird bei der destillativen Abtrennung des Ketons auch ein Teil des Opfer-Alkohols und zum Teil auch noch Teile der wässrigen Phase mit entfernt. Diese Destillationsverluste werden in der Regel durch Nachdosieren des Opfer-Alkohols und ggf. des Wassers ausgeglichen. Die Destillationsraten liegen in einem Bereich von 0,02%/min bis 2%/min, bevorzugt von 0,05%/min bis 1 %/min bezogen auf das Reaktionsvolumen. Die Manteltemperaturen des Reaktors liegen zwischen 5-70 Kelvin, bevorzugt zwischen 10-40 Kelvin über der Reaktorinnentemperatur.

Die Destillation wird besonders gut in einem Druckbereich von 1-500 mbar, bevorzugt 10-200 mbar durchgeführt.

Ebenfalls offenbart ist die Umsetzung der Verbindung der allgemeinen Formel II, wie z.B. der Formel IV, in Gegenwart eines Mikroorganismus erfolgen zu lassen, der ausgewählt ist unter Bakterien der Familien Enterobacteriaceae, Pseudomonadaceae, Rhizobiaceae, Lactobacillaceae, Streptomycetaceae, Rhodococcaceae und Nocardiaceae.. Der Mikroorganismus kann insbesondere ein rekombinanter Mikroorganismus sein, der mit einem Nukleinsäurekonstrukt transformiert ist, welches für ein Enzym mit Dehydrogenase-Aktivität gemäß obiger Definition kodiert.

Ebenfalls offenbart ist
- einen ein Enzym mit Dehydrogenase-Aktivität produzierenden Mikroorganismus aus einer natürlichen Quelle zu isolieren oder rekombinant herzustellen,
- diesen Mikroorganismus zu vermehren,
- aus dem Mikroorganismus das Enzym mit Dehydrogenase-Aktivität gegebenenfalls zu isolieren oder eine dieses Enzym enthaltende Proteinfraktion herzustellen, und
- den Mikroorganismus gemäß Stufe b) oder das Enzym gemäß Stufe c) in ein Medium zu überführen, das eine Verbindung der Formel I enthält.

Gegenstand der Offenlegung sind außerdem kodierende Nukleinsäuresequenzen, umfassend die kodierende Sequenz für ein Polypeptid gemäß obiger Definition.

Weiterhin betrifft die Offenlegung Expressionskassetten, umfassend in operativer Verknüpfung mit wenigstens einer regulativen Nukleinsäuresequenz eine kodierende Nukleinsäuresequenz gemäß obiger Definition.

Ein weiterer Gegenstand der Offenlegung sind rekombinante Vektoren, umfassend wenigstens eine solche Expressionskassette.

Die Offenlegung betrifft auch prokaryotische oder eukaryotische Wirte, welche mit wenigstens einem erfindungsgemäßen Vektor transformiert sind.

Ein letzter Gegenstand der Offenlegung betrifft die Verwendung eines Enzyms mit Dehydrogenase-Aktivität gemäß obiger Definition oder eines dieses Enzym produzierenden Mikroorganismus zur Herstellung von Verbindungen der Formeln I oder III, und deren Weiterverarbeitung beispielsweise zur Herstellung von Duloxetine (Formel VIII)

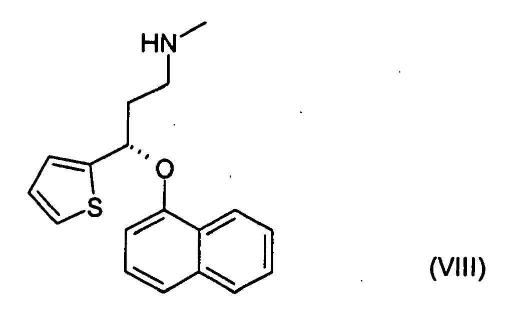

### Detaillierte Beschreibung der Erfindung:

### A. Allgemeine Begriffe und Definitionen

Werden keine andere Angaben gemacht, so gelten folgende allgemeine Bedeutungen:
"Halogen" steht für Fluor, Chlor, Brom oder Jod , insbesondere Fluor oder Chlor.
"Niedrigalkyl" steht für geradkettige oder verzweigte Alkylreste 1 bis 6 Atomen, wie Methyl, Ethyl, i- oder n-Propyl, n-, i-, sec.- oder tert.-Butyl, n-Pentyl oder 2-MethylButyl, n-Hexyl, 2-Methyl-pentyl, 3-Methyl-pently, 2-Ethyl-butyl.
"Niedrigalkenyl" steht für die ein- oder mehrfach, vorzugsweise einfach oder zweifach ungesättigten Analoga oben genannter Alkylreste mit 2 bis 6 Kohlenstoffatomen, wobei die Doppelbindung in beliebiger Position der Kohlenstoffkette liegen kann.
"Niedrigalkoxy" steht für die Sauerstoff-terminierten Analoga obiger Alkylreste.
"Aryl" steht für einen ein- oder mehrkernigen, vorzugsweise ein- oder zweikernigen, gegebenenfalls substituierten aromatischen Rest, insbesondere für Phenyl oder für ein über eine beliebige Ringposition gebundenes Naphthyl, wie 1- oder 2-Naphthyl. Diese Arylreste können gegebenenfalls 1 oder 2 gleiche oder verschiedene Substituenten tragen, ausgewählt unter Halogen, Niedrigalkyl, Niedrigalkoxy gemäß obiger Definition oder Trifluormethyl.

Substituierte Alkanone, (S)-Alkanole und Derivate davon

Erfindungsgemäß durch enzymatische Katalyse zugängliche Alkanole sind solche obiger Formel (I) worin
- n: für einen ganzzahligen Wert von 0 bis 5 steht;
- Cyc: für einen gegebenenfalls substituierten, ein- oder mehrkernigen, gesättigten oder ungesättigten, carbocyclischen oder heterocyclischen Ring steht, und
- R¹: für Halogen, SH, OH, NO₂, NR²R³ oder NR²R³R⁴⁺X⁻ steht, wobei R², R³ und R⁴ unabhängig voneinander für H oder einen Niedrigalkyl- oder Niedrigalkoxy-Rest stehen und X⁻ für ein Gegenion steht.

Die zur enzymatischen Synthese verwendeten Alkanone obiger Formel II sind an sich bekannte Verbindungen und unter Anwendung allgemein bekannter organischer Syntheseverfahren zugänglich (vgl. z.B. EP-A- 0 273 658).

Vorzugsweise steht n in obigen Verbindungen für 0, 1 oder 2, insbesondere für 1.

Als Beispiele für carbo- und heterocyclische Gruppen Cyc sind insbesondere ein- oder zweikernigen, vorzugsweise einkernige, Gruppen mit bis zu 4, vorzugsweise 1 oder 2 gleichen oder verschiedenen Ring-Heteroatomen, ausgewählt unter O, N und S sind zu nennen:
Diese carbo- oder heterocyclischen Ringe umfassen insbesondere 3 bis 12, vorzugsweise 4, 5 oder 6 Ring-Kohlenstoffatomen. Als Beispiele können genannt werden Cyclopropyl, Cyclobutyl, Cyclopenty, Cyclohexyl, Cycloheptyl, die ein- oder mehrfach ungesättigten Analoga davon, wie Cyclobutenyl, Cyclopentenyl, Cyclohexenyl, Cycloheptenyl, Cyclohexadienyl, Cycloheptadienyl; sowie 5- bis 7-gliedrige gesättigte oder ein oder mehrfach ungesättigte heterocyclische Reste mit 1 bis 4 Heteroatomen, die ausgewählt sind unter O, N und S, wobei der Heterocyclus gegebenenfalls mit einem weiteren Heterocyclus oder Carbocyclus kondensiert sein kann. Insbesondere sind zu nennen heterocyclische Reste, abgeleitet von Pyrrolidin, Tetrahydrofuran, Piperidin, Morpholin, Pyrrol, Furan, Thiophen, Pyrazol, Imidazol, Oxazol, Thiazol, Pyridin, Pyran, Pyrimidin, Pyridazin, Pyrazin, Cumaron, Indol und Chinolin.
Die Reste Cyc können dabei über eine beliebige Ringposition, vorzugsweise über ein Ring-Kohlenstoffatom, an das Alkanon bzw. das Alkanol gebunden sein.
Beispiele für geeignet Cyc-Reste sind 2-Thienyl, 3-Thienyl; 2-Furanyl, 3-Furanyl; 2-Pyridyl, 3-Pyridyl oder 4-Pyridyl; 2-Thiazolyl, 4-Thiazolyl oder 5-Thiazolyl; 4-Methyl-2-thienyl, 3-Ethyl-2-thienyl, 2-Methyl-3-thienyl, 4-Propyl-3-thienyl, 5-n-Butyl-2-thienyl, 4-Methyl-3-thienyl, 3-Methyl-2-thienyl; 3-Chlor-2-thienyl, 4-Brom-3-thienyl, 2-lod-3-thienyl, 5-lod-3-thienyl, 4-Fluor-2-thienyl, 2-Brom-3-thienyl, und 4-Chlor-2-thienyl.
Die Reste Cyc können weiterhin ein- oder mehrfach, wie z.B. ein- oder zweifach, substituiert sein. Vorzugsweise sitzen die Substituenten an einem Ring-Kohlenstoffatom. Beispiele für geeignete Substituenten sind Halogen, Niedrigalkyl, Niedrigalkenyl, Niedrigalkoxy, -OH, -SH, -NO₂ oder NR²R³, wobei R² und R³ obige Bedeutungen besitzen, bevorzugt Halogen oder Niedrigalkyl.

R¹ steht insbesondere für Halogen, NR²R³ oder NR²R³R⁴⁺X⁻, wobei R², R³ bzw. R², R³ und R⁴ unabhängig voneinander für H oder einen Niedrigalkyl- oder Niedrigalkoxy-Rest stehen und X⁻ für ein Gegenion steht, wobei vorzugsweise einer der Reste R², R³ und R⁴ für H steht. Geeignete Gegenionen sind beispielsweise Säureanionen, wie sie beispielsweise bei Herstellung eines Säureadditionssalzes anfallen. Beispiel hierzu sind z.B. in der EP-A-0 273 658 genannt, worauf hiermit Bezug genommen wird. Bevorzugte Beispiele für Reste R¹ sind insbesondere Fluor oder Chlor, sowie NR²R³ worin R² und R³ gleich oder verschieden sind und für H oder Methyl, Ethyl oder n-Propyl stehen; besonders bevorzugt steht R¹ für Chlor oder -NHMethyl.

### C. Geeignete Enzyme mit Dehydrogenase-Aktivität

Bevorzugte Enzyme mit Dehydrogenase-Aktivität umfassen eine Aminosäuresequenz gemäß SEQ ID NO: 2.

Ebenfalls offenbart sind "funktionale Äquivalente" der konkret offenbarten Enzyme mit Dehydrogenase-Aktivität und die Verwendung dieser in den er-Verfahren.

"Funktionale Äquivalente" oder Analoga der konkret offenbarten Enzyme sind im Rahmen der vorliegenden Erfindung davon verschiedene Polypeptide, welche weiterhin die gewünschte biologische Aktivität, wie z.B. Substratspezifität, besitzen. So versteht man beispielsweise unter "funktionalen Äquivalenten" Enzyme, die von 3-Chlor-1-(thien-2-yl)-propan-1-on zum entsprechenden S-Alkohol reduzieren und die mindestens 50 %, bevorzugt 60 %, besonders bevorzugt 75 %, ganz besonders bevorzugt 90 % der Aktivität eines Enzyms mit der in SEQ ID NO:2 aufgeführten Aminosäuresequenz aufweist. Funktionale Äquivalente sind außerdem vorzugsweise zwischen pH 4 bis 10 stabil und besitzen vorteilhaft ein pH-Optimum zwischen pH 5 und 8 sowie ein Temperaturoptimum im Bereich von 20°C bis 80°C.

Unter "funktionalen Äquivalenten" versteht man insbesondere auch Mutanten, welche in wenigstens einer Sequenzposition der oben genannten Aminosäuresequenzen eine andere als die konkret genannte Aminosäure aufweisen aber trotzdem eine der oben genannten biologischen Aktivitäten besitzen. "Funktionale Äquivalente" umfassen somit die durch eine oder mehrere Aminosäure-Additionen, - Substitutionen, -Deletionen und/oder -Inversionen erhältlichen Mutanten, wobei die genannten Veränderungen in jeglicher Sequenzposition auftreten können, solange sie zu einer Mutante mit dem Eigenschaftsprofil führen. Funktionale Äquivalenz ist insbesondere auch dann gegeben, wenn die Reaktivitätsmuster zwischen Mutante und unverändertem Polypeptid qualitativ übereinstimmen, d.h. beispielsweise gleiche Substrate mit unterschiedlicher Geschwindigkeit umgesetzt werden. Beispiele für geeignete Aminosäuresubstitutionen sind folgender Tabelle zu entnehmen:

| Ursprünglicher Rest | Beispiele der Substitution |
|---|---|
| Ala | Ser |
| Arg | Lys |
| Asn | Gln; His |
| Asp | Glu |
| Cys | Ser |
| Gln | Asn |
| Glu | Asp |
| Gly | Pro |
| His | Asn; Gln |
| Ile | Leu; Val |
| Leu | Ile; Val |
| Lys | Arg ; Gln ; Glu |
| Met | Leu; lle |
| Phe | Met ; Leu ; Tyr |
| Ser | Thr |
| Thr | Ser |
| Trp | Tyr |
| Tyr | Trp ; Phe |
| Val | Ile; Leu |

Unter "funktionalen Äquivalenten" versteht man insbesondere auch Mutanten, welche in wenigstens einer Sequenzposition der oben genannten Aminosäuresequenzen eine andere als die konkret genannte Aminosäure aufweisen aber trotzdem eine der oben genannten biologischen Aktivitäten besitzen. "Funktionale Äquivalente" umfassen somit die durch eine oder mehrere Aminosäure-Additionen, - Substitutionen, -Deletionen und/oder -Inversionen erhältlichen Mutanten, wobei die genannten Veränderungen in jeglicher Sequenzposition auftreten können, solange sie zu einer Mutante mit dem Eigenschaftsprofil führen. Funktionale Äquivalenz ist insbesondere auch dann gegeben, wenn die Reaktivitätsmuster zwischen Mutante und unverändertem Polypeptid qualitativ übereinstimmen, d.h. beispielsweise gleiche Substrate mit unterschiedlicher Geschwindigkeit umgesetzt werden.

"Funktionale Äquivalente" im obigen Sinne sind auch "Präkursoren" der beschriebenen Polypeptide sowie "funktionale Derivate" und "Salze" der Polypeptide.

"Präkursoren" sind dabei natürliche oder synthetische Vorstufen der Polypeptide mit oder ohne der gewünschten biologischen Aktiviät.

Unter dem Ausdruck "Salze" versteht man sowohl Salze von Carboxylgruppen als auch Säureadditionssalze von Aminogruppen der Proteinmoleküle. Salze von Carboxygruppen können in an sich bekannter Weise hergestellt werden und umfassen anorganische Salze, wie zum Beispiel Natrium-, Calcium-, Ammonium-, Eisen- und Zinksalze, sowie Salze mit organischen Basen, wie zum Beispiel Aminen, wie Triethanolamin, Arginin, Lysin, Piperidin und dergleichen. Säureadditionssalze, wie zum Beispiel Salze mit Mineralsäuren, wie Salzsäure oder Schwefelsäure und Salze mit organischen Säuren, wie Essigsäure und Oxalsäure sind ebenfalls Gegenstand der Offenlegung.

"Funktionale Derivate" der Polypeptide können an funktionellen Aminosäure-Seitengruppen oder an deren N- oder C-terminalen Ende mit Hilfe bekannter Techniken ebenfalls hergestellt werden. Derartige Derivate umfassen beispielsweise aliphatische Ester von Carbonsäuregruppen, Amide von Carbonsäuregruppen, erhältlich durch Umsetzung mit Ammoniak oder mit einem primären oder sekundären Amin; N-Acylderivate freier Aminogruppen, hergestellt durch Umsetzung mit Acylgruppen; oder O-Acylderivate freier Hydroxygruppen, hergestellt durch Umsetzung mit Acylgruppen.

"Funktionale Äquivalente" umfassen natürlich auch Polypeptide welche aus anderen Organismen zugänglich sind, sowie natürlich vorkommende Varianten. Beispielsweise lassen sich durch Sequenzvergleich Bereiche homologer Sequenzregionen festlegen und in Anlehnung an die konkreten Vorgaben der Erfindung äquivalente Enzyme ermitteln.

"Funktionale Äquivalente" umfassen ebenfalls Fragmente, vorzugsweise einzelne Domänen oder Sequenzmotive, der erfindungsgemäßen Polypeptide, welche z.B. die gewünschte biologische Funktion aufweisen.

"Funktionale Äquivalente" sind außerdem Fusionsproteine, welche eine der oben genannten Polypeptidsequenzen oder davon abgeleitete funktionale Äquivalente und wenigstens eine weitere, davon funktionell verschiedene, heterologe Sequenz in funktioneller N- oder C-terminaler Verknüpfung (d.h. ohne gegenseitigen wesentliche funktionelle Beeinträchtigung der Fusionsproteinteile) aufweisen. Nichtlimitierende Beispiele für derartige heterologe Sequenzen sind z.B. Signalpeptide oder Enzyme.

Offenbart sind "funktionale Äquivalente", Homologe zu den konkret offenbarten Proteinen. Diese besitzen wenigstens 60 %, vorzugsweise wenigstens 75% ins besondere wenigsten 85 %, wie z.B. 90%, 95% oder 99%, Homologie zu einer der konkret offenbarten Aminosäuresequenzen, berechnet nach dem Algorithmus von Pearson und Lipman, Proc. Natl. Acad, Sci. (USA) 85(8), 1988, 2444-2448. Eine prozentuale Homologie eines homologen Polypeptids bedeutet insbesondere prozentuale Identität der Aminosäurereste bezogen auf die Gesamtlänge einer der hierin konkret beschriebenen Aminosäuresequenzen.

Im Falle einer möglichen Proteinglykosylierung umfassen "funktionale Äquivalente" Proteine des oben bezeichneten Typs in deglykosylierter bzw. glykosylierter Form sowie durch Veränderung des Glykosylierungsmusters erhältliche abgewandelte Formen.

Homologe der Proteine oder Polypeptide können durch Mutagenese erzeugt werden, z.B. durch Punktmutation oder Verkürzung des Proteins.

Homologe der Proteine können durch Screening kombinatorischer Banken von Mutanten, wie z.B. Verkürzungsmutanten, identifiziert werden. Beispielsweise kann eine variegierte Bank von Protein-Varianten durch kombinatorische Mutagenese auf Nukleinsäureebene erzeugt werden, wie z.B. durch enzymatisches Ligieren eines Gemisches synthetischer Oligonukleotide. Es gibt eine Vielzahl von Verfahren, die zur Herstellung von Banken potentieller Homologer aus einer degenerierten Oligonukleotidsequenz verwendet werden können. Die chemische Synthese einer degenerierten Gensequenz kann in einem DNA-Syntheseautomaten durchgeführt werden, und das synthetische Gen kann dann in einen geeigneten Expressionsvektor ligiert werden. Die Verwendung eines degenerierten Gensatzes ermöglicht die Bereitstellung sämtlicher Sequenzen in einem Gemisch, die den gewünschten Satz an potentiellen Proteinsequenzen kodieren. Verfahren zur Synthese degenerierter Oligonukleotide sind dem Fachmann bekannt (z.B. Narang, S.A. (1983) Tetrahedron 39:3; Itakura et al. (1984) Annu. Rev. Biochem. 53:323; Itakura et al., (1984) Science 198:1056; Ike et al. (1983) Nucleic Acids Res. 11:477).

Im Stand der Technik sind mehrere Techniken zum Screening von Genprodukten kombinatorischer Banken, die durch Punktmutationen oder Verkürzung hergestellt worden sind, und zum Screening von cDNA-Banken auf Genprodukte mit einer ausgewählten Eigenschaft bekannt. Diese Techniken lassen sich an das schnelle Screening der Genbanken anpassen, die durch kombinatorische Mutagenese erfindungsgemäßer Homologer erzeugt worden sind. Die am häufigsten verwendeten Techniken zum Screening großer Genbanken, die einer Analyse mit hohem Durchsatz unterliegen, umfassen das Klonieren der Genbank in replizierbare Expressionsvektoren, Transformieren der geeigneten Zellen mit der resultierenden Vektorenbank und Exprimieren der kombinatorischen Gene unter Bedingungen, unter denen der Nachweis der gewünschten Aktivität die Isolation des Vektors, der das Gen kodiert, dessen Produkt nachgewiesen wurde, erleichtert. Recursive-Ensemble-Mutagenese (REM), eine Technik, die die Häufigkeit funktioneller Mutanten in den Banken vergrößert, kann in Kombination mit den Screeningtests verwendet werden, um Homologe zu identifizieren (Arkin und Yourvan (1992) PNAS 89:7811-7815; Delgrave et al. (1993) Protein Engineering 6(3):327-331).

### D. Für die Dehydrogenasen kodierende Nukleinsäuresequenzen

Gegenstand der Offenlegung sind insbesondere Nukleinsäuresequenzen (einzel- und doppelsträngige DNA- und RNA-Sequenzen, wie z.B. cDNA und mRNA), die für ein Enzym mit Dehydrogenase-Aktivität kodieren. Bevorzugt sind Nukleinsäuresequenzen, welche z.B. für Aminosäuresequenzen gemäß SEQ ID NO:2 oder charakteristische Teilsequenzen davon kodieren, oder Nukleinsäuresequenzen gemäß SEQ ID NO:1 oder charakteristische Teilsequenzen davon umfassen.

Alle hierin erwähnten Nukleinsäuresequenzen sind in an sich bekannter Weise durch chemische Synthese aus den Nukleotidbausteinen, wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seiten 896-897) erfolgen. Die Anlagerung synthetischer Oligonukleotide und Auffüllen von Lücken mit Hilfe des Klenow-Fragmentes der DNA-Polymerase und Ligationsreaktionen sowie allgemeine Klonierungsverfahren werden in Sambrook et al. (1989), Molecular Cloning: A laboratory manual, Cold Spring Harbor Laboratory Press, beschrieben.

Gegenstand der Offenlegung sind auch Nukleinsäuresequenzen (einzel- und doppelsträngige DNA- und RNA-Sequenzen, wie z.B. cDNA und mRNA), kodierend für eines der obigen Polypeptide und deren funktionalen Äquivalenten, welche z.B. unter Verwendung künstlicher Nukleotidanaloga zugänglich sind.

Die Offenlegung betrifft sowohl isolierte Nukleinsäuremoleküle, welche für Polypeptide bzw. Proteine oder biologisch aktive Abschnitte davon kodieren, als auch Nukleinsäurefragmente, die z.B. zur Verwendung als Hybridisierungssonden oder Primer zur Identifizierung oder Amplifizierung von kodierenden Nukleinsäuren verwendet werden können.

Die Nukleinsäuremoleküle können zudem untranslatierte Sequenzen vom 3'- und/oder 5'-Ende des kodierenden Genbereichs enthalten

Die Erfindung umfasst weiterhin die zu den konkret beschriebenen Nukleotidsequenzen komplementären Nukleinsäuremoleküle oder einen Abschnitt davon.

Die Nukleotidsequenzen ermöglichen die Erzeugung von Sonden und Primern, die zur Identifizierung und/oder Klonierung von homologer Sequenzen in anderen Zelltypen und Organismen verwendbar sind. Solche Sonden bzw. Primer umfassen gewöhnlich einen Nukleotidsequenzbereich, der unter "stringenten" Bedingungen (siehe unten) an mindestens etwa 12, vorzugsweise mindestens etwa 25, wie z.B. etwa 40, 50 oder 75 aufeinanderfolgende Nukleotide eines Sense-Stranges einer Nukleinsäuresequenz oder eines entsprechenden Antisense-Stranges hybridisiert.

Ein "isoliertes" Nukleinsäuremolekül wird von anderen Nukleinsäuremolekülen abgetrennt, die in der natürlichen Quelle der Nukleinsäure zugegen sind und kann überdies im wesentlichen frei von anderem zellulären Material oder Kulturmedium sein, wenn es durch rekombinante Techniken hergestellt wird, oder frei von chemischen Vorstufen oder anderen Chemikalien sein, wenn es chemisch synthetisiert wird.

Ein Nukleinsäuremolekül kann mittels molekularbiologischer Standard-Techniken und der bereitgestellten Sequenzinformation isoliert werden. Beispielsweise kann cDNA aus einer geeigneten cDNA-Bank isoliert werden, indem eine der konkret offenbarten vollständigen Sequenzen oder ein Abschnitt davon als Hybridisierungssonde und Standard-Hybridisierungstechniken (wie z.B. beschrieben in Sambrook, J., Fritsch, E.F. und Maniatis, T. Molecular Cloning: A Laboratory Manual. 2. Aufl., Cold Spring Harbor Laboratory, Cold Spring Harbor Laboratory Press, Cold Spring Harbor, NY, 1989) verwendet werden. Überdies lässt sich ein Nukleinsäuremolekül, umfassend eine der offenbarten Sequenzen oder ein Abschnitt davon, durch Polymerasekettenreaktion isolieren, wobei die Oligonukleotidprimer, die auf der Basis dieser Sequenz erstellt wurden, verwendet werden. Die so amplifizierte Nukleinsäure kann in einen geeigneten Vektor kloniert werden und durch DNA-Sequenzanalyse charakterisiert werden. Die Oligonukleotide können ferner durch Standard-Syntheseverfahren, z.B. mit einem automatischen DNA-Synthesegerät, hergestellt werden.

Die Nukleinsäuresequenzen lassen sich prinzipiell aus allen Organismen identifizieren und isolieren. Vorteilhaft lassen sich die Nukleinsäuresequenzen oder die Homologen davon, aus Pilzen, Hefen, Archeen oder Bakterien isolieren. Als Bakterien seien gram-negative und gram-positive Bakterien genannt. Bevorzugt werden die Nukleinsäuren aus gram-negativen Bakterien vorteilhaft aus α-Proteobakterien, β-Proteobakterien oder γ-Proteobakterien, besonders bevorzugt aus Bakterien der Ordnungen der *Burkholderiales, Hydrogenophilales, Methylophilales, Neisseriales, Nitrosomonadales, Procabacteriales* oder *Rhodocyclales*. Ganz besonders bevorzugt aus Bakterien der Familie der Rhodocyclaceae. Insbesondere bevorzugt aus den Gattung *Azoarcus.* Insbesondere bevorzugt aus Arten *Azoarcus anaerobius, Azoarcus buckelii, Azoarcus communis, Azoarcus evansii, Azoarcus indigens, Azoarcus toluclasticus, Azoarcus tolulyticus, Azoarcus toluvorans, Azoarcus* sp., *Azoarcus* sp. 22Lin, *Azoarcus* sp. BH72, *Azoarcus* sp. CC-11, *Azoarcus* sp. CIB, *Azoarcus* sp. CR23, *Azoarcus* sp. EB1, *Azoarcus* sp. EbN1, *Azoarcus* sp. FL05, *Azoarcus* sp. HA, *Azoarcus* sp. HxN1, *Azoarcus* sp. mXyN1, *Azoarcus* sp. PbN1, *Azoarcus* sp. PH002, *Azoarcus* sp. T und *Azoarcus* sp. ToN1.

Besonders bevorzugt verwendet man Dehydrogenasen aus *Azoarcus* sp EbN1.

Nukleinsäuresequenzen lassen sich beispielsweise mit üblichen Hybridisierungsverfahren oder der PCR-Technik aus anderen Organismen, z.B. über genomische oder cDNA-Banken, isolieren. Diese DNA-Sequenzen hybridisieren unter Standardbedingungen mit den Sequenzen. Zur Hybridisierung werden vorteilhaft kurze Oligonukleotide der konservierten Bereiche beispielsweise aus dem aktiven Zentrum, die über Vergleiche mit einer Dehydrogenase in dem Fachmann bekannter Weise ermittelt werden können, verwendet. Es können aber auch längere Fragmente der Nukleinsäuren oder die vollständigen Sequenzen für die Hybridisierung verwendet werden. Je nach der verwendeten Nukleinsäure (Oligonukleotid, längeres Fragment oder vollständige Sequenz) oder je nachdem welche Nukleinsäureart DNA oder RNA für die Hybridisierung verwendet werden, variieren diese Standardbedingungen. So liegen beispielsweise die Schmelztemperaturen für DNA:DNA-Hybride ca 10 °C niedriger als die von DNA:RNA-Hybriden gleicher Länge.

Unter Standardbedingungen sind beispielsweise je nach Nukleinsäure Temperaturen zwischen 42 und 58 °C in einer wäßrigen Pufferlösung mit einer Konzentration zwischen 0,1 bis 5 x SSC (1 X SSC = 0,15 M NaCl, 15 mM Natriumcitrat, pH 7,2) oder zusätzlich in Gegenwart von 50% Formamid wie beispielsweise 42 °C in 5 x SSC, 50% Formamid zu verstehen. Vorteilhafterweise liegen die Hybridisierungsbedingungen für DNA:DNA-Hybride bei 0,1 x SSC und Temperaturen zwischen etwa 20 °C bis 45 °C, bevorzugt zwischen etwa 30 °C bis 45 °C. Für DNA:RNA-Hybride liegen die Hybridisierungsbedingungen vorteilhaft bei 0,1 x SSC und Temperaturen zwischen etwa 30 °C bis 55 °C, bevorzugt zwischen etwa 45 °C bis 55 °C. Diese angegebenen Temperaturen für die Hybridisierung sind beispielhaft kalkulierte Schmelztemperaturwerte für eine Nukleinsäure mit einer Länge von ca. 100 Nukleotiden und einem G + C-Gehalt von 50 % in Abwesenheit von Formamid. Die experimentellen Bedingungen für die DNA-Hybridisierung sind in einschlägigen Lehrbüchern der Genetik, wie beispielsweise Sambrook et al., "Molecular Cloning", Cold Spring Harbor Laboratory, 1989, beschrieben und lassen sich nach dem Fachmann bekannten Formeln beispielsweise abhängig von der Länge der Nukleinsäuren, der Art der Hybride oder dem G + C-Gehalt berechnen. Weitere Informationen zur Hybridisierung kann der Fachmann folgenden Lehrbüchern entnehmen: Ausubel et al. (eds), 1985, Current Protocols in Molecular Biology, John Wiley & Sons, New York; Hames and Higgins (eds), 1985, Nucleic Acids Hybridization: A Practical Approach, IRL Press at Oxford University Press, Oxford; Brown (ed), 1991, Essential Molecular Biology: A Practical Approach, IRL Press at Oxford University Press, Oxford.

Gegenstand der Offenlegung sind auch Derivate der konkret offenbarten oder ableitbaren Nukleinsäuresequenzen.

So können weitere Nukleinsäuresequenzen von SEQ ID NO:1 abgeleitet sein und sich davon durch Addition, Substitution, Insertion oder Deletion einzelner oder mehrerer Nukleotide unterscheiden, aber weiterhin für Polypeptide mit dem gewünschten Eigenschaftsprofil kodieren.

Offenbart sind auch solche Nukleinsäuresequenzen, die sogenannte stumme Mutationen umfassen oder entsprechend der Codon-Nutzung eins speziellen Ursprungs- oder Wirtsorganismus, im Vergleich zu einer konkret genannten Sequenz verändert sind, ebenso wie natürlich vorkommende Varianten, wie z.B. Spleißvarianten oder Allelvarianten, davon.

Gegenstand sind ebenso durch konservative Nukleotidsubstutionen (d.h. die betreffende Aminosäure wird durch eine Aminosäure gleicher Ladung, Größe, Polarität und/oder Löslichkeit ersetzt) erhältliche Sequenzen.

Gegenstand der offenlegung sind auch die durch Sequenzpolymorphismen von den konkret offenbarten Nukleinsäuren abgeleiteten Moleküle. Diese genetischen Polymorphismen können zwischen Individuen innerhalb einer Population aufgrund der natürlichen Variation existieren. Diese natürlichen Variationen bewirken üblicherweise eine Varianz von 1 bis 5 % in der Nukleotidsequenz eines Gens.

Unter Derivaten einer Nukleinsäuresequenz sind beispielsweise Allelvarianten zu verstehen, die mindestens 40 % Homologie auf der abgeleiteten Aminosäureebene, bevorzugt mindestens 60 % Homologie, ganz besonders bevorzugt mindestens 80, 85, 90, 93, 95 oder 98 % Homologie über den gesamten Sequenzbereich aufweisen (bezüglich Homologie auf Aminosäureebene sei auf obige Ausführungen zu den Polypeptiden verwiesen auf). Über Teilbereiche der Sequenzen können die Homologien vorteilhaft höher liegen.

Weiterhin sind unter Derivaten auch Homologe der Nukleinsäuresequenzen beispielsweise pilzliche oder bakterielle Homologe, verkürzte Sequenzen, Einzelstrang-DNA oder RNA der kodierenden und nichtkodierenden DNA-Sequenz, zu verstehen. So besitzten z.B. auf DNA-Ebene eine Homologie von mindestens 40 %, bevorzugt von mindestens 60 %, besonders bevorzugt von mindestens 70 %, ganz besonders bevorzugt von mindestens 80 % über den gesamten angegebenen DNA-Bereich.

Außerdem sind unter Derivaten beispielsweise Fusionen mit Promotoren zu verstehen. Die Promotoren, die den angegebenen Nukleotidsequenzen vorgeschalten sind, können durch ein oder mehrere Nukleotidaustausche, Insertionen, Inversionen und/oder Deletionen verändert sein, ohne dass aber die Funktionalität bzw. Wirksamkeit der Promotoren beeinträchtigt sind. Des weiteren können die Promotoren durch Veränderung ihrer Sequenz in ihrer Wirksamkeit erhöht oder komplett durch wirksamere Promotoren auch artfremder Organismen ausgetauscht werden.

Unter Derivaten sind auch Varianten zu verstehen, deren Nukleotidsequenz im Bereich von -1 bis -1000 Basen stromaufwärts vor dem Startkodon oder 0 bis 1000 Basen stromabwärts nach dem Stopkodon so verändert wurden, dass die Genexpression und/oder die Proteinexpression verändert, bevorzugt erhöht wird.

Weiterhin umfasst die Offenlegung auch Nukleinsäuresequenzen, welchen mit oben genannten kodierenden Sequenzen unter "stringenten Bedingungen" hybridisieren. Diese Polynukleotide lassen sich bei der Durchmusterung von genomischen oder cDNA-Banken auffinden und gegebenenfalls daraus mit geeigneten Primern mittels PCR vermehren und anschließend beispielsweise mit geeigneten Sonden isolieren. Darüber hinaus können Polynukleotide auch auf chemischem Wege synthetisiert werden. Unter dieser Eigenschaft versteht man die Fähigkeit eines Poly- oder Oligonukleotids unter stringenten Bedingungen an eine nahezu komplementäre Sequenz zu binden, während unter diesen Bedingungen unspezifische Bindungen zwischen nicht-komplementären Partnern unterbleiben. Dazu sollten die Sequenzen zu 70-100%, vorzugsweise zu 90-100%, komplementär sein. Die Eigenschaft komplementärer Sequenzen, spezifisch aneinander binden zu können, macht man sich beispielsweise in der Northern- oder Southern-Blot-Technik oder bei der Primerbindung in PCR oder RT-PCR zunutze. Üblicherweise werden dazu Oligonukleotide ab einer Länge von 30 Basenpaaren eingesetzt. Unter stringenten Bedingungen versteht man beispielsweise in der Northern-Blot-Technik die Verwendung einer 50 - 70 °C, vorzugsweise 60 - 65 °C warmen Waschlösung, beispielsweise 0,1x SSC-Puffer mit 0,1% SDS (20x SSC: 3M NaCl, 0,3M Na-Citrat, pH 7,0) zur Elution unspezifisch hybridisierter cDNA-Sonden oder Oligonukleotide. Dabei bleiben, wie oben erwähnt, nur in hohem Maße komplementäre Nukleinsäuren aneinander gebunden. Die Einstellung stringenter Bedingungen ist dem Fachmann bekannt und ist z:B. in Ausubel et al., Current Protocols in Molecular Biology, John Wiley & Sons, N.Y. (1989), 6.3.1-6.3.6. beschrieben.

### E. Ausgestaltungen der Konstrukte

Gegenstand der offenlegung sind außerdem Expressionskonstrukte, enthaltend unter der genetischen Kontrolle regulativer Nukleinsäuresequenzen eine für ein Polypeptid kodierende Nukleinsäuresequenz; sowie Vektoren, umfassend wenigstens eines dieser Expressionskonstrukte.

Vorzugsweise umfassen solche Konstrukte 5'-stromaufwärts von der jeweiligen kodierenden Sequenz einen Promotor und 3'-stromabwärts eine Terminatorsequenz sowie gegebenenfalls weitere übliche regulative Elemente, und zwar jeweils operativ verknüpft mit der kodierenden Sequenz.

Unter einer "operativen Verknüpfung" versteht man die sequentielle Anordnung von Promotor, kodierender Sequenz, Terminator und gegebenenfalls weiterer regulativer Elemente derart, dass jedes der regulativen Elemente seine Funktion bei der Expression der kodierenden Sequenz bestimmungsgemäß erfüllen kann. Beispiele für operativ verknüpfbare Sequenzen sind Targeting-Sequenzen sowie Enhancer, Polyadenylierungssignale und dergleichen. Weitere regulative Elemente umfassen selektierbare Marker, Amplifikationssignale, Replikationsursprünge und dergleichen. Geeignete regulatorische Sequenzen sind z.B. beschrieben in Goeddel, Gene Expression Technology: Methods in Enzymology 185, Academic Press, San Diego, CA (1990).

Unter einem Nukleinsäurekonstrukt sind insbesondere solche zu verstehen, bei weichen das Gen für eine Dehydrogenase mit einem oder mehreren Regulationssignalen zur Steuerung, z.B. Erhöhung, der Genexpression operativ oder funktionell verknüpft wurden.

Zusätzlich zu diesen Regulationssequenzen kann die natürliche Regulation dieser Sequenzen vor den eigentlichen Strukturgenen noch vorhanden sein und gegebenenfalls genetisch verändert worden sein, so dass die natürliche Regulation ausgeschaltet und die Expression der Gene erhöht wurde. Das Nukleinsäurekonstrukt kann aber auch einfacher aufgebaut sein, das heißt es wurden keine zusätzlichen Regulationssignale vor die kodierende Sequenz insertiert und der natürliche Promotor mit seiner Regulation wurde nicht entfernt. Stattdessen wird die natürliche Regulationssequenz so mutiert, dass keine Regulation mehr erfolgt und die Genexpression gesteigert wird.

Ein bevorzugtes Nukleinsäurekonstrukt enthält vorteilhafterweise auch eine oder mehrere der schon erwähnten "Enhancer" Sequenzen, funktionell verknüpft mit dem Promotor, die eine erhöhte Expression der Nukleinsäuresequenz ermöglichen. Auch am 3'-Ende der DNA-Sequenzen können zusätzliche vorteilhafte Sequenzen inseriert werden, wie weitere regulatorische Elemente oder Terminatoren. Die Nukleinsäuren können in einer oder mehreren Kopien im Konstrukt enthalten sein. Im Konstrukt können noch weitere Marker, wie Antibiotikaresistenzen oder Auxotrophien komplementierende Gene, gegebenenfalls zur Selektion auf das Konstrukt enthalten sein.

Vorteilhafte Regulationssequenzen für das Verfahren sind beispielsweise in Promotoren wie cos-, tac-, trp-, tet-, trp-tet-, Ipp-, Iac-, Ipp-Iac-, Iacl^{q-,} T7-, T5-, T3-, gal-, trc-, ara-, rhaP (rhaP_{BAD})SP6-, Iambda-P_{R}- oder im Iambda-P_{L}-Promotor enthalten, die vorteilhafterweise in gram-negativen Bakterien Anwendung finden. Weitere vorteilhafte Regulationssequenzen sind beispielsweise in den gram-positiven Promotoren amy und SPO2, in den Hefe- oder Pilzpromotoren ADC1, MFalpha , AC, P-60, CYC1, GAPDH, TEF, rp28, ADH enthalten. In diesem Zusammenhang sind auch die Promotoren der Pyruvatdecarboxylase und der Methanoloxidase, beispielsweiseaus Hansenula vorteilhaft. Es können auch künstliche Promotoren für die Regulation verwendet werden.

Das Nukleinsäurekonstrukt wird zur Expression in einem Wirtsorganismus vorteilhafterweise in einen Vektor, wie beispielsweise einem Plasmid oder einem Phagen inseriert, der eine optimale Expression der Gene im Wirt ermöglicht. Unter Vektoren sind außer Plasmiden und Phagen auch alle anderen dem Fachmann bekannten Vektoren, also z.B. Viren, wie SV40, CMV, Baculovirus und Adenovirus, Transposons, IS-Elemente, Phasmide, Cosmide, und lineare oder zirkuläre DNA zu verstehen. Diese Vektoren können autonom im Wirtsorganismus repliziert oder chromosomal repliziert werden. Diese Vektoren stellen eine weitere Ausgestaltung der Offenlegung dar. Geeignete Plasmide sind beispielsweise in E. coli pLG338, pACYC184, pBR322, pUC18, pUC19, pKC30, pRep4, pHS1, pKK223-3, pDHE19.2, pHS2, pPLc236, pMBL24, pLG200, pUR290, pIN-III¹¹³-B1, Igt11 oder pBdCl, in Streptomyces plJ101, plJ364, po702 oder plJ361, in Bacillus pUB110, pC194 oder pBD214, in Corynebacterium pSA77 oder pAJ667, in Pilzen pALS1, plL2 oder pBB116, in Hefen 2alphaM, pAG-1, YEp6, YEp13 oder pEMBLYe23 oder in Pflanzen pLGV23, pGHlac⁺, pBIN19, pAK2004 oder pDH51. Die genannten Plasmide stellen eine kleine Auswahl der möglichen Plasmide dar. Weitere Plasmide sind dem Fachmann wohl bekannt und können beispielsweise aus dem Buch Cloning Vectors (Eds. Pouwels P. H. et al. Elsevier, Amsterdam-New York-Oxford, 1985 , ISBN 0 444 904018) entnommen werden.

Vorteilhafterweise enthält das Nukleinsäurekonstrukt zur Expression der weiteren enthaltenen Gene zusätzlich noch 3'- und/oder 5'-terminale regulatorische Sequenzen zur Steigerung der Expression, die je nach ausgewähltem Wirtorganismus und Gen oder Gene für eine optimale Expression ausgewählt werden.

Diese regulatorischen Sequenzen sollen die gezielte Expression der Gene und der Proteinexpression ermöglichen. Dies kann beispielsweise je nach Wirtsorganismus bedeuten, dass das Gen erst nach Induktion exprimiert oder überexprimiert wird, oder dass es sofort exprimiert und/oder überexprimiert wird.

Die regulatorischen Sequenzen bzw. Faktoren können dabei vorzugsweise die Genexpression der eingeführten Gene positiv beeinflussen und dadurch erhöhen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird.

In einer weiteren Ausgestaltungsform des Vektors kann der das Nukleinsäurekonstrukt oder die Nukleinsäure enthaltende Vektor auch vorteilhafterweise in Form einer linearen DNA in die Mikroorganismen eingeführt werden und über heterologe oder homologe Rekombination in das Genom des Wirtsorganismus integriert werden. Diese lineare DNA kann aus einem linearisierten Vektor wie einem Plasmid oder nur aus dem Nukleinsäurekonstrukt oder der Nukleinsäure bestehen.

Für eine optimale Expression heterologer Gene in Organismen ist es vorteilhaft die Nukleinsäuresequenzen entsprechend des im Organismus verwendeten spezifischen "codon usage" zu verändern. Der "codon usage" läßt sich anhand von Computerauswertungen anderer, bekannter Gene des betreffenden Organismus leicht ermitteln.

Die Herstellung einer Expressionskassette erfolgt durch Fusion eines geeigneten Promotors mit einer geeigneten kodierenden Nukleotidsequenz sowie einem Terminator- oder Polyadenylierungssignal. Dazu verwendet man gängige Rekombinations- und Klonierungstechniken, wie sie beispielsweise in T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley Interscience (1987) beschrieben sind.

Das rekombinante Nukleinsäurekonstrukt bzw. Genkonstrukt wird zur Expression in einem geeigneten Wirtsorganismus vorteilhafterweise in einen wirtsspezifischen Vektor insertiert, der eine optimale Expression der Gene im Wirt ermöglicht. Vektoren sind dem Fachmann wohl bekannt und können beispielsweise aus "Cloning Vectors" (Pouwels P. H. et al., Hrsg, Elsevier, Amsterdam-New York-Oxford, 1985) entnommen werden.

### F. Brauchbare Wirtsorganismen

Mit Hilfe der Vektoren oder Konstrukte sind rekombinante Mikroorganismen herstellbar, welche beispielsweise mit wenigstens einem Vektor transformiert sind und zur Produktion der Polypeptide eingesetzt werden können. Vorteilhafterweise werden die oben beschriebenen rekombinanten Konstrukte in ein geeignetes Wirtssystem eingebracht und exprimiert. Dabei werden vorzugsweise dem Fachmann bekannte geläufige Klonierungs- und Transfektionsmethoden, wie beispielsweise Co-Präzipitation, Protoplastenfusion, Elektroporation, retrovirale Transfektion und dergleichen, verwendet, um die genannten Nukleinsäuren im jeweiligen Expressionssystem zur Expression zu bringen. Geeignete Systeme werden beispielsweise in Current Protocols in Molecular Biology, F. Ausubel et al., Hrsg., Wiley Interscience, New York 1997, oder Sambrook et al. Molecular Cloning: A Laboratory Manual. 2. Aufl., Cold Spring Harbor Laboratory, Cold Spring Harbor Laboratory Press, Cold Spring Harbor, NY, 1989 beschrieben.

Es sind auch homolog rekombinierte Mikroorganismen herstellbar. Dazu wird ein Vektor hergestellt, der zumindest einen Abschnitt eines Gens oder einer kodierenden Sequenz enthält, worin gegebenenfalls wenigstens eine Aminosäure-Deletion, -Addition oder -Substitution eingebracht worden ist, um die Sequenz zu verändern, z.B. funktionell zu disrumpieren ("Knockout"-Vektor). Die eingebrachte Sequenz kann z.B. auch ein Homologes aus einem verwandten Mikroorganismus sein oder aus einer Säugetier-, Hefe- oder Insektenquelle abgeleitet sein. Der zur homologen Rekombination verwendete Vektor kann alternativ derart ausgestaltet sein, daß das endogene Gen bei homologer Rekombination mutiert oder anderweitig verändert ist, jedoch noch das funktionelle Protein kodiert (z.B. kann der stromaufwärts gelegene regulatorische Bereich derart verändert sein, dass dadurch die Expression des endogenen Proteins verändert wird). Der veränderte Abschnitt des Gens ist im homologen Rekombinationsvektor. Die Konstruktion geeigneter Vektoren zur homologen Rekombination ist z.B. beschrieben in Thomas, K.R. und Capecchi, M.R. (1987) Cell 51:503.

Als rekombinante Wirtsorganismen für die Nukleinsäure oder dem Nukleinsäurekonstrukt kommen prinzipiell alle prokaryontischen oder eukaryontischen Organismen in Frage. Vorteilhafterweise werden als Wirtsorganismen Mikroorganismen wie Bakterien, Pilze oder Hefen verwendet. Vorteilhaft werden gram-positive oder gram-negative Bakterien, bevorzugt Bakterien der Familien Enterobacteriaceae, Pseudomonadaceae, Rhizobiaceae, Streptomycetaceae oder Nocardiaceae, besonders bevorzugt Bakterien der Gattungen Escherichia, Pseudomonas, Streptomyces, Nocardia, Burkholderia, Salmonella, Agrobacterium oder Rhodococcus verwendet.

Ganz besonders bevorzugt ist die Gattung und Art Escherichia coli. Weitere vorteilhafte Bakterien sind darüber hinaus in der Gruppe der alpha-Proteobacterien, beta-Proteobacterien oder gamma-Proteobacterien zu finden.

Der Wirtsorganismus oder die Wirtsorganismen gemäß der Offenlegung enthalten dabei vorzugsweise mindestens eine der in dieser Offenlegung beschriebenen Nukleinsäuresequenzen, Nukleinsäurekonstrukte oder Vektoren, die für ein Enzym mit Dehydrogenaseaktivität kodieren.

Die im Verfahren verwendeten Organismen werden je nach Wirtsorganismus in dem Fachmann bekannter Weise angezogen bzw. gezüchtet. Mikroorganismen werden in der Regel in einem flüssigen Medium, das eine Kohlenstoffquelle meist in Form von Zuckern, eine Stickstoffquelle meist in Form von organischen Stickstoffquellen wie Hefeextrakt oder Salzen wie Ammoniumsulfat, Spurenelemente wie Eisen-, Mangan-, Magnesiumsalze und gegebenenfalls Vitamine enthält, bei Temperaturen zwischen 0 °C und 100 °C, bevorzugt zwischen 10 °C bis 60 °C unter Sauerstoffbegasung angezogen. Dabei kann der pH der Nährflüssigkeit auf einen festen Wert gehalten werden, das heißt während der Anzucht reguliert werden oder nicht. Die Anzucht kann "batch"-weise, "semi batch"-weise oder kontinuierlich erfolgen. Nährstoffe können zu beginn der Fermentation vorgelegt oder semikontinuierlich oder kontinuierlich nachgefüttert werden. Das Keton kann direkt zur Anzucht gegeben werden oder vorteilhaft nach Anzucht. Die Enzyme können nach dem in den Beispielen beschriebenen Verfahren aus den Organismen isoliert werden oder als Rohextrakt für die Reaktion verwendet werden.

### G. Rekombinante Herstellung der Polypeptide

Gegenstand der Offenlegung sind weiterhin Verfahren zur rekombinanten Herstellung Polypeptide oder funktioneller, biologisch aktiver Fragmente davon,
wobei man einen Polypeptide-produzierenden Mikroorganismus kultiviert, gegebenenfalls die Expression der Polypeptide induziert und diese aus der Kultur isoliert. Die Polypeptide können so auch in großtechnischem Maßstab produziert werden, falls dies erwünscht ist.

Der rekombinante Mikroorganismus kann nach bekannten Verfahren kultiviert und fermentiert werden. Bakterien können beispielsweise in TB- oder LB-Medium und bei einer Temperatur von 20 bis 40°C und einem pH-Wert von 6 bis 9 vermehrt werden. Im Einzelnen werden geeignete Kultivierungsbedingungen beispielsweise in T. Maniatis, E.F. Fritsch and J. Sambrook, Molecular Cloning: A Laboratory Manual, Cold Spring Harbor Laboratory, Cold Spring Harbor, NY (1989) beschrieben.

Die Zellen werden dann, falls die Polypeptide nicht in das Kulturmedium sezerniert werden, aufgeschlossen und das Produkt nach bekannten Proteinisolierungsverfahren aus dem Lysat gewonnen. Die Zellen können wahlweise durch hochfrequenten Ultraschall, durch hohen Druck, wie z.B. in einer French-Druckzelle, durch Osmolyse, durch Einwirkung von Detergenzien, lytischen Enzymen oder organischen Lösungsmitteln, durch Homogenisatoren oder durch Kombination mehrerer der aufgeführten Verfahren aufgeschlossen werden.

Eine Aufreinigung der Polypeptide kann mit bekannten, chromatographischen Verfahren erzielt werden, wie Molekularsieb-Chromatographie (Gelfiltration), wie Q-Sepharose-Chromatographie, lonenaustausch-Chromatographie und hydrophobe Chromatographie, sowie mit anderen üblichen Verfahren wie Ultrafiltration, Kristallisation, Aussalzen, Dialyse und nativer Gelelektrophorese. Geeignete Verfahren werden beispielsweise in Cooper, F. G., Biochemische Arbeitsmethoden, Verlag Walter de Gruyter, Berlin, New York oder in Scopes, R., Protein Purification, Springer Verlag, New York, Heidelberg, Berlin beschrieben.

Vorteilhaft kann es sein, zur Isolierung des rekombinanten Proteins Vektorsysteme oder Oligonukleotide zu verwenden, die die cDNA um bestimmte Nukleotidsequenzen verlängern und damit für veränderte Polypeptide oder Fusionsproteine kodieren, die z.B. einer einfacheren Reinigung dienen. Derartige geeignete Modifikationen sind beispielsweise als Anker fungierende sogenannte "Tags", wie z.B. die als Hexa-Histidin-Anker bekannte Modifikation oder Epitope, die als Antigene von Antikörpern erkannt werden können (beschrieben zum Beispiel in Harlow, E. and Lane, D., 1988, Antibodies: A Laboratory Manual. Cold Spring Harbor (N.Y.) Press). Diese Anker können zur Anheftung der Proteine an einen festen Träger, wie z.B. einer Polymermatrix, dienen, die beispielsweise in einer Chromatographiesäule eingefüllt sein kann, oder an einer Mikrotiterplatte oder an einem sonstigen Träger verwendet werden kann.

Gleichzeitig können diese Anker auch zur Erkennung der Proteine verwendet werden. Zur Erkennung der Proteine können außerdem übliche Marker, wie Fluoreszenzfarbstoffe, Enzymmarker, die nach Reaktion mit einem Substrat ein detektierbares Reaktionsprodukt bilden, oder radioaktive Marker, allein oder in Kombination mit den Ankern zur Derivatisierung der Proteine verwendet werden.

### H. Durchführung des erfindungsgemäßen Verfahrens zur Herstellung von (S)-Alkanole

Die verwendeten Enzyme mit Dehydrogenase-Aktivität können im erfindungsgemäßen Verfahren als freies oder immobilisiertes Enzym verwendet werden.

Das erfindungsgemäße Verfahren wird vorteilhaft bei einer Temperatur zwischen 0 °C bis 95 °C, bevorzugt zwischen 10 °C bis 85 °C, besonders bevorzugt zwischen 15 °C bis 75 °C durchgeführt.

Der pH-Wert im erfindungsgemäßen Verfahren wird vorteilhaft zwischen pH 4 und 12, bevorzugt zwischen pH 4,5 und 9, besonders bevorzugt zwischen pH 5 und 8 gehalten.

Unter enantiomerenreinen bzw. chiralen Produkten bzw. optisch aktiven Alkoholen sind im erfindungsgemäßen Verfahren Enantiomere zu verstehen, die eine Enantiomerenanreicherung zeigen. Bevorzugt werden im Verfahren Enantiomerenreinheiten von mindestens 70 %ee, bevorzugt von min. 80 %ee, besonders bevorzugt von min. 90 %ee, ganz besonders bevorzugt min. 98 %ee erreicht.

Für das erfindungsgemäße Verfahren können wachsende Zellen verwendet werden, die die Nukleinsäuren, Nukleinsäurekonstrukte oder Vektoren enthalten. Auch ruhende oder aufgeschlossene Zellen können verwendet werden. Unter aufgeschlossenen Zellen sind beispielsweise Zellen zu verstehen, die über eine Behandlung mit beispielsweise Lösungsmitteln durchlässig gemacht worden sind, oder Zellen die über eine Enzymbehandlung, über eine mechanische Behandlung (z.B. French Press oder Ultraschall) oder über eine sonstige Methode aufgebrochen wurden. Die so erhaltenen Rohextrakte sind für das erfindungsgemäße Verfahren vorteilhaft geeignet. Auch gereinigte oder angereinigte Enzyme können für das Verfahren verwendet werden. Ebenfalls geeignet sind immobilisierte Mikroorganismen oder Enzyme, die vorteilhaft in der Reaktion Anwendung finden können.

Werden für das erfindungsgemäße Verfahren freie Organismen oder Enzyme verwendet, so werden diese vor der Extraktion zweckmäßigerweise abgetrennt beispielsweise über eine Filtration oder Zentrifugation.

Das im erfindungsgemäßen Verfahren hergestellte Produkt, beispielsweise (1S)-3-Methylamino-1-(2-thienyl)-propan-1-ol, lässt sich vorteilhaft aus der wässrigen Reaktionslösung über Extraktion oder Destillation gewinnen. Die Extraktion kann zur Erhöhung der Ausbeute mehrfach wiederholt werden. Beispiele für geeignete Extraktionsmittel sind Lösungsmittel, wie Toluol, Methylenchlorid, Butylacetat, Diisopropylether, Benzol, MTBE oder Essigester, ohne darauf beschränkt zu sein. Alternativ kann das im erfindungsgemäßen Verfahren hergestellte Produkt, beispielsweise (1S)-3-Methylamino-1-(2-thienyl)-propan-1-ol, sich vorteilhaft aus der organischen Phase der Reaktionslösung über Extraktion oder Destillation oder/und Kristallisation gewinnen. Die Extraktion kann zur Erhöhung der Ausbeute mehrfach wiederholt werden. Beispiele für geeignete Extraktionsmittel sind Lösungsmittel, wie Toluol, Methylenchlorid, Butylacetat, Diisopropylether, Benzol, MTBE oder Essigester, ohne darauf beschränkt zu sein.

Nach Einengen der organischen Phase können die Produkte in der Regel in guten chemischen Reinheiten, das heißt größer 80 % chemische Reinheit, gewonnen werden. Nach Extraktion kann die organische Phase mit dem Produkt aber auch nur zum Teil eingeengt werden und das Produkt auskristallisiert werden. Dazu wird die Lösung vorteilhaft auf eine Temperatur von 0 °C bis 10 °C abgekühlt. Die Kristallisation kann auch direkt aus der organischen Lösung oder aus einer wässrigen Lösung erfolgen. Das auskristallisierte Produkt kann nochmals im gleichen oder in einem anderen Lösungsmittel zur erneuten Kristallisation aufgenommen werden und nochmals kristallisiert werden. Durch die anschließende vorteilhafte mindestens einmalig durchgeführte Kristallisation kann die Enantiomerenreinheit des Produktes falls erforderlich weiter gesteigert werden.

Bei den genannten Aufarbeitungsarten lässt sich das Produkt des erfindungsgemäßen Verfahrens in Ausbeuten von 60 bis 100 %, bevorzugt von 80 bis 100 %, besonders bevorzugt von 90 bis 100 %, bezogen auf das für die Reaktion eingesetzte Substrat, wie z.B. von 3-Methylamino-1-(2-thienyl)-propan-1-on, isolieren. Das isolierte Produkt zeichnet sich durch eine hohe chemische Reinheit von > 90 %, bevorzugt > 95 % besonders bevorzugt von > 98 % aus. Weiterhin haben die Produkt eine hohe Enantiomerenreinheit, die vorteilhaft falls erforderlich durch die Kristallisation weiter gesteigert werden kann.

Das erfindungsgemäße Verfahren kann batchweise, semi-batchweise oder kontinuierlich betrieben werden.

Die Durchführung des Verfahrens kann vorteilhafterweise in Bioreaktoren erfolgen, wie z.B. beschrieben in Biotechnology, Band 3, 2. Auflage, Rehm et al Hrsg., (1993) insbesondere Kapitel II.

Die obige Beschreibung und die nachstehenden Beispiele dienen der Verdeutlichung der Erfindung.

Der Vorteil des erfindungsgemäßen Verfahrens liegt in der besonders hohen Ausbeute des optisch aktiven Alkanols der Formel (I) bzw. der nahezu quantitativen Umwandlung von Alkanon (II).

### Experimenteller Teil:

### Beispiel 1: Klonierung der Phenylethanol-Dehydrogenase aus Azoarcus sp EbN1 mittels synthetischer Oligonukleotide.

Die Sequenz des Phenylethanol-Dehydrogenasegens aus *Azoarcus* sp EbN1 ist in Datenbanken hinterlegt (Genbank ID 25956124, Region:25073 bis 25822). Von der Nukleinsäuresequenz des Phenylethanol-Dehydrogenase-Gens wurden Oligonukleotide abgeleitet, aus denen nach bekannten Verfahren das Gen des synthetisiert wurde. Die DNA-Sequenz der Oligonukleotide ist in Tabelle 1 zusammengefasst. Abbildung 3 zeigt die Position der einzelnen Oligonukleotide zum gesamten Phenylethanol-Dehydrogenase-Gen aus *Azoarcus* sp EbN1. Verfahren zur Herstellung von synthetischer Gene sind beispielsweise bei Y.P. Shi, P. Das, B. Holloway, V. Udhayakumar, J.E.Tongren, F. Candal, S. Biswas, R. Ahmad, S.E. Hasnain und A.A. Lal, Vaccine 2000, 18, S. 2902-2914 beschrieben. Die erhaltene Sequenz ist mit der publizierten Sequenz identisch.

Das PCR-Produkt wurde mit den Restriktionsendonukleasen Ndel und BamHI verdaut und in entsprechend verdauten pDHE19.2-Vektor (DE19848129) kloniert. Die Ligationsansätze wurden in E.coli XL1 Blue (Stratagene) transformiert. Das Plasmid pDHEEbN1 wurde in den Stamm *E.coli* TG10 pAgro4 pHSG575 transformiert (TG10: ein RhaA⁻-Derivat von E.coli TG1(Stratagene); pAgro4: Takeshita, S; Sato, M; Toba, M; Masahashi, W; Hashimoto-Gotoh, T (1987) Gene 61, 63-74; pHSG575: T. Tomoyasu et al (2001), Mol. Microbiol. 40(2), 397-413).

Die rekombianten *E. coli* werden mit E. coli LU 11558 bezeichnet.

**Tabelle 1**

| | Oligonukleotide zur Herstellung des synthetischen Phenylethanol-Dehydrogenase-Gens aus *Azoarcus* sp EbN1 |
|---|---|
| #1 | GAGCGATTTGCGGTCGAAGGTGCCGACATCGCAATCGCGGATCTGGTGCCGGCCCCGGAAGCCGAGGCAGCAATCAGGAACCTCGGTCGGCGCGT |
| #2 | GCACCTTCGACCGCAAATCGCTCCGCAATTGCCCGCCCGATGCCGTTGGCACCGCCGGTAATTACTGCAAGCTTGTCCTTCAGTCTTTGCGTCAT |
| #3 | AAACGTGGAGATGACCTGCTTTCCGAATGCTTCTACGTCGCCAGGTTGCGAGACATCGCACTTCACGGTCAGAACGCGCCGACCGAGGTTCCTGA |
| #4 | GGACCTGGGGAAGGACGGAATCACTGTTAACGCCATCGCGCCGAGCCTTGTCCGCACGGCAACAACCGAAGCTTCTGCATTGTCCGCGATGTTCGAC |
| #5 | AAAGCAGGTCATCTCCACGTTTGGTCGCTGCGACATCCTCGTCAACAACGCGGGAATTTACCCGCTGATTCCTTTTGACGAGCTGACCTTTGAAC |
| #6 | AGGAACGCAGCTGCGCCCGTCAGATCCAGGGGCACCTGAAGACGCGGAATCGCCTGAAGCATGTTTGGCAGCACGTCGAACATCGCGGACAATGCAG |
| #7 | GATTCCGTCCTTCCCGAGGTCCGAGGCAAGGGCGCGGGTAAAGCCTATGTTTGCCGCTTTCGTGCTGATGTAATGGGTATACGCCTCGATCTTTAGC |
| #8 | GAAGGCTTTTGTCCCCGGGATGAAGAGGAACGGGTGGGGACGCATCATCAACCTGACTTCGACGACATATTGGCTAAAGATCGAGGCGTATACCC |
| #9 | CATCCCGGGGACAAAAGCCTTCGCCATAAGAAAACGTGAATCGACGTTGATCTCGAATGTTTTCTTCCACTGTTCAAAGGTCAGCTCGTCAAAAG |
| #10 | GACGGGCGCAGCTGCGTTCCTGGCTTCCGATGACGCCAGTTTTATTACAGGCCAGACGCTCGCGGTTGATGGCGGTATGGTGAGACACTGA |
| #11 | TCAGTGTCTCACCATACCGCCATC |
| #12 | ATGACGCAAAGACTGAAGGACAAG |

### Beispiel 2: Klonierung der Phenylethanol-Dehydrogenase aus Azoarcus sp EbN1 mittels PCR

Die Sequenz des Phenylethanol-Dehydrogenasegens aus *Azoarcus* sp EbN1 ist in Datenbanken hinterlegt (Genbank ID 25956124, Region 25073 bis 25822). Von der Nukleinsäuresequenz des Phenylethanol-Dehydrogenase-Gens wurden Oligonukleotide abgeleitet (Primer MKe0387 und MKe0388), die wie folgt der Klonierung des Dehydrogenase-Gens durch PCR-Amplifikation dienten.

### PCR:

| Template | Primer | Produktlänge |
|---|---|---|
| subklonierte DNA aus *Azoarcus* sp EbN1 oder chromosomale DNA aus *Azoarcus* sp EbN1 | MKe0387 und MKe0388 | ca. 750 bp |

### Primer:

| Primer Nr. | Sequenz (5'->3') | Position |
|---|---|---|
| MKe0387 | GGGAATTCCATATGACGCAAAGACTGAAGG | N-Term-Primer |
| Mke0388 | CGCGGATCCTCAGTGTCTCACCATACCGCC | C-Term-Primer |

Je 130 ng der Primer MKe0387 und MKe0388 wurden equimolar gemischt. Die PCR wurde nach Stratagene-Standardvorschrift mit PfuTurbo-Polymerase (Roche Applied Science) und dem folgenden Temperatur-Gradienten-Programm durchgeführt: 95°C für 5 min; 30 Zyklen mit 95°C für 45 sec, 55°C für 45 sec, und 72°C für 1 min; 72°C für 10 min.; 10°C bis zur Verwendung. Das PCR-Produkt (-0,75 kB) wurde durch Agarosegel-Elektrophorese (1,2%E-Gel, Invitrogen) und Säulen-Chromatographie (GFX-Kit, Amersham Pharmacia) isoliert und anschließend sequenziert (Sequenzierprimer:
MKe0387 und MKe0388). Die erhaltene Sequenz ist mit der publizierten Sequenz identisch.
Das PCR-Produkt wurde mit den Restriktionsendonukleasen Ndel und BamHI verdaut und in entsprechend verdauten pDHE19.2-Vektor (DE19848129) kloniert. Die Ligationsansätze wurden in E.coli XL1 Blue (Stratagene) transformiert. Die Sequenzierung entsprechender Klone ergab als Insert im so erhaltenen Plasmid pDHEEbN1 die in SEQ ID NO:1 dargestellte Nukleinsäuresequenz,
Das Plasmid pDHEEbN1 wurde in den Stamm E.coli TG10 pAgro4 pHSG575 transformiert (TG10: ein RhaA⁻-Derivat von E.coli TG1 (Stratagene); pAgro4: Takeshita, S; Sato, M; Toba, M; Masahashi, W; Hashimoto-Gotoh, T (1987) Gene 61, 63-74; pHSG575: T. Tomoyasu et al (2001), Mol. Microbiol. 40(2), 397-413). Die rekombianten E. coli werden mit E.coli LU 11558 bezeichnet.

### Beispiel 3: Bereitstellung rekombinanter Phenylethanol-Dehydrogenase aus E.coli LU 11558

E.coli LU 11558 wurden in 20mL LB-Amp/SpeGCm (100µg/l Ampicillin; 100µg/l Spectinomycin; 20µg/l Chloramphenicol), 0,1mM IPTG, 0,5g/L Rhamnose in 100mL Erlenmeyerkolben (Schikanen) 18 h bei 37°C angezogen, bei 5000*g/10min zentrifugiert, einmal mit 10mM TRIS*HCl, pH7,0 gewaschen und in 2 mL des gleichen Puffers resuspendiert.

Zellfreier Proteinrohextrakt wurde hergestellt indem Zellpaste von E.coli LU 11558 mit 0,7ml Glaskugeln (d=0,5mm) in einer Schwingmühle (3x 5min mit Zwischenkühlung auf Eis) aufgeschlossen wurde.

### Beispiel 4: Aktivitätsbestimmung der rekombinanten Dehydrogenase aus E.coli LU 11558

Je 6 Transformanten wurden in 20mL LBAmp/Spec/Cm (100µg/l Amp; 100mg/ISpec; 20µg/ICm) 0,1mM IPTG 0,5g/L Rhamnose in 100mL Erlenmeyerkolben (Schikanen) 18 h bei 37°C angezogen, bei 5000*g/10min zentrifugiert, einmal mit 10mM Tris/HCl pH7,0 gewaschen und in 2 mL des gleichen Puffers resuspendiert. 100 mL Zellsuspension wurden 20 min in 900 µl 50mM MES pH6 mit 50µl/ml Glucose-DH, 100mM Glucose, 100mM NaCl, 1 mM NADH, 1 mM NADPH und mit 10 mM 3-Chlor-1-(thien-2-yl)-propan-1-on schüttelnd inkubiert. Die Ansätze wurden analog zu Beispiel 4 analysiert. Im Durchschnitt wurden 0,13 mM 3-Chlor-1-(thien-2-yl)-propan-1-ol gebildet, was einer Aktivität von 6,6 U/L Kultursuspension entspricht. In analogen Ansätzen mit Rohextrakt, der durch Zellaufschluß mit 0,7ml Glaskugeln (d=0,5mm) in einer Schwingmühle (3x 5min mit Zwischenkühlung auf Eis) gewonnen wurde, wurden 0,21 mM 3-Chlor-1-(thien-2-yl)-propan-1-ol, entsprechend einer Aktivität von 10,7 U/L, gemessen. In Kontrollversuchen ohne Zusatz von Rhamnose bei der Anzucht war kein 3-Chlor-1-(thien-2-yl)-propan-1-ol detektierbar.

### Beispiel 5 Analytik von 3-Chlor-1-(thien-2-yl)-propan-1-on und 3-Chlor-1-(thien-2-yl)-propan-1-ol

Die Konzentration von 3-Chlor-1-(thien-2-yl)-propan-1-on und 3-Chlor-1-(thien-2-yl)-propan-1-ol lassen sich mittels HPLC bestimmen. Je nach Wahl der stationären und mobilen Phase lassen sich neben der Konzentration auch ee-Wert bestimmen.

### a) achirale Analytik

Die Quantifizierung der Umsetzung wurde mit folgendem System durchgeführt:

| | |
|---|---|
| stationäre Phase: | Chromoltih SpeedROD RP18, 50*4, 6µm, Merck (Darmstadt) temperiert auf 45°C |
| mobilePhase: | Laufmittel A: 10mM KH₂PO₄, pH 2.5 |
| | Laufmittel B: Acetonitril |
| | Gradient: 0-0.5 min, 35%B; 0.5-1.0 min 35 auf 80%B; 1.0-1.2 min 80%B; 1.2-1.3 min 80% - 35%B; 1.3-2.0 min 35%B; |
| Flussrate: | 1.5 ml/min |
| Detektion: | UV-Detektion bei 230 und 260nm |
| Retentionszeiten: | 3-Chlor-1-(thien-2-yl)-propan-1-on: ca. 1.6 min |
| | 3-Chlor-1-(thien-2-yl)-propan-1-ol: ca. 1.3 min |

Mit authentischem Material wird eine Eichreihe erstellt, anhand derer die Konzentration unbekannter Proben bestimmt werden kann.

### b) chirale Analytik

| | |
|---|---|
| stationäre Phase: | Chiracel OD-H, 250*4, 6µm, Daicel, temperiert auf 40°C |
| mobilePhase: | Laufmittel A: n-Hexan |
| | Laufmittel B: iso-Propanol isokratisch mit 2.5% B |
| Flussrate: | 1.0 ml/min |
| Detektion: | UV-Detektion bei 230 und 260nm |
| Retentionszeiten: | 3-Chlor-1-(thien-2-yl)-propan-1-on: ca. 9.5 min |
| | (1S)-3-Chlor-1-(thien-2-yl)-propan-1-ol: ca. 16.6 min |
| | (1R)-3-Chlor-1-(thien-2-yl)-propan-1-ol: ca. 18.3 min |

Mit authentischem Material wird eine Eichreihe erstellt, anhand derer die Konzentration unbekannter Proben bestimmt werden kann.

### Beispiel 6: Aktivitätsbestimmung der rekombinanten Dehydrogenase aus E.coli LU 11558

Je 6 Transformanten von E.coli LU 11558 wurden in 20mL LBAmp/Spec/Cm (100µg/l Amp; 50mg/ISpec; 10µg/ICm) 0,1mM IPTG 0,5g/L Rhamnose in 100mL Erlenmeyerkolben (Schikanen) 18 h bei 37°C angezogen, bei 5000*g/10min zentrifugiert, einmal mit 10mM Tris/HCl pH7,0 gewaschen und in 2 mL des gleichen Puffers resuspendiert. 100 mL Zellsuspension wurden 20 min in 900µl 50mM MES pH6 mit 50µl/ml Glucose-DH (Beispiel.....), 100mM Glucose, 100mM NaCl, 1 mM NADH, 1 mM NADPH und mit 10 mM 3-Chlor-1-(thien-2-yl)-propan-1-on schüttelnd inkubiert. Die Ansätze wurden analog zu Beispiel 4 analysiert. Im Durchschnitt wurden 0,13 mM 3-Chlor-1-(thien-2-yl)-propan-1-ol gebildet, was einer Aktivität von 6,6 U/L Kultursuspension entspricht. In analogen Ansätzen mit Rohextrakt, der durch Zellaufschluß mit 0,7ml Glaskugeln (d=0,5mm) in einer Schwingmühle (3x 5min mit Zwischenkühlung auf Eis) gewonnen wurde, wurden 0,21 mM 3-Chlor-1-(thien-2-yl)-propan-1-ol, entsprechend einer Aktivität von 10,7 U/L, gemessen. In Kontrollversuchen ohne Zusatz von Rhamnose bei der Anzucht war kein 3-Chlor-1-(thien-2-yl)-propan-1-ol detektierbar.

### Beispiel 7: Herstellung von (S)-3-Chlor-1-(thien-2-yl)-propan-1-ol) mit der rekombinanten Dehydrogenase aus Azoarcus sp EbN1 mit 2-Pentanol

Ruhende Zellen von E.coli LU 11558 (1-20 g/L Biomasse) wurden mit 0,2 mM NAD⁺ und 185 mM 3-Chlor-1-(thien-2-yl)-propan-1-on (12,5 ml) in 221 ml 50mM KH₂PO₄, und 250 ml 2-Pentanol bei 40 °C im 0,75 L-Reaktor gerührt (250 rpm). Die Reaktion wurde durch Titration mit 5 M NaOH bei pH 5,5 gehalten. Durch HPLC-Analytik wurde die Reaktion verfolgt bis zu einer Konzentration von 50-350 mM TACA in der organischen Phase.

### Beispiel 8: Herstellung von (S)-3-Chlor-1-(thien-2-yl)-propan-1-ol) mit der rekombinanten Dehydrogenase aus Azoarcus sp EbN1 mit 2-Pentanol

Ruhende Zellen von E.coli LU 11558 (1-20 g/L Biomasse) wurden mit 0,2 mM NAD⁺ und 370 mM 3-Chlor-1-(thien-2-yl)-propan-1-on (200 ml) in 1432 ml 50mM KH₂PO₄, und 2000 ml 2-Pentanol bei 40 °C im 4 L-Reaktor gerührt (250 rpm). Die Reaktion wurde durch Titration mit 5 M NaOH bei pH 5,5 gehalten. Durch HPLC-Analytik wurde die Reaktion verfolgt bis zu einer Konzentration von 100-650 mM TACA in der organischen Phase.

### Beispiel 9: Herstellung von (S)-3-Chlor-1-(thien-2-yl)-propan-1-ol) mit der rekombinanten Dehydrogenase aus Azoarcus sp EbN1 mit 2-Pentanol

Ruhende Zellen von E.coli LU 11558 (1-20 g/L Biomasse) wurden mit 0,2 mM NAD⁺ und 370 mM 3-Chlor-1-(thien-2-yl)-propan-1-on (200 ml) in 1432 ml 50mM KH₂PO₄, und 2000 ml 2-Pentanol bei 40 °C im 4 L-Reaktor gerührt (250 rpm). Die Reaktion wurde durch Titration mit 5 M NaOH bei pH 5,5 gehalten. Es wurde ein Vakuum von ca. 85 mbar und eine Manteltemperatur von 50-70 °C angelegt. Die bei Destillationsraten von 2-10 ml/min erhaltenen Destillatmengen an Wasser und 2-Pentanol wurden während der Reaktion fortlaufend wieder zugegeben. Durch HPLC-Analytik wurde die Reaktion verfolgt bis zu einer Konzentration von 300-700 mM TACA in der organischen Phase. Das 3-Chlor-1-(thien-2-yl)-propan-1-on wurde bis auf 0-30% Restanteil umgesetzt.

### Beispiel 10: Herstellung von (S)-3-Chlor-1-(thien-2-yl)-propan-1-ol) mit der rekombinanten Dehydrogenase aus Azoarcus sp EbN1 mit 2-Pentanol

Ruhende Zellen von E.coli LU 11558 (1-20 g/L Biomasse) wurden mit 0,2 mM NAD⁺ und 200 mM 3-Chlor-1-(thien-2-yl)-propan-1-on (12,5 ml) in 221 ml 50mM KH₂PO₄, und 250 ml 2-Pentanol bei 40 °C im 0,75 L-Reaktor gerührt (250 rpm). Die Reaktion wurde durch Titration mit 5 M NaOH bei pH 5,5 gehalten. Durch HPLC-Analytik wurde die Reaktion verfolgt bis zu einer Konzentration von 50-320 mM TACA in der organischen Phase.

### Abbildung

SEQ ID NO:1 Nukleinsäuresäuresequenz der Phenylethanol-Dehydrogenase aus
Azoarcus sp EbN1 (Genbank ID 25956124, Region:25073 bis 25822)

1 atgacgcaaa gactgaagga caagcttgca gtaattaccg gcggtgccaa cggcatcggg
61 cgggcaattg cggagcgatt tgcggtcgaa ggtgccgaca tcgcaatcgc ggatctggtg
121 ccggccccgg aagccgaggc agcaatcagg aacctcggtc ggcgcgttct gaccgtgaag
181 tgcgatgtct cgcaacctgg cgacgtagaa gcattcggaa agcaggtcat ctccacgttt
241 ggtcgctgcg acatcctcgt caacaacgcg ggaatttacc cgctgattcc ttttgacgag
301 ctgacctttg aacagtggaa gaaaacattc gagatcaacg tcgattcagg ttttcttatg
361 gcgaaggctt ttgtccccgg gatgaagagg aacgggtggg gacgcatcat caacctgact
421 tcgacgacat attggctaaa gatcgaggcg tatacccatt acatcagcac gaaagcggca
481 aacataggct ttacccgcgc ccttgcctcg gacctgggga aggacggaat cactgttaac
541 gccatcgcgc cgagccttgt ccgcacggca acaaccgaag cttctgcatt gtccgcgatg
601 ttcgacgtgc tgccaaacat gcttcaggcg attccgcgtc ttcaggtgcc cctggatctg
661 acgggcgcag ctgcgttcct ggcttccgat gacgccagtt ttattacagg ccagacgctc
721 gcggttgatg gcggtatggt gagacactga

SEQ ID NO:2 Aminosäuresequenz der Phenylethanol-Dehydrogenase aus Azoarcus
sp EbN1 accession (Genbank protein ID CAD58337)

1 MTQRLKDKLA VITGGANGIG RAIAERFAVE GADIAIADLV PAPEAEAAIR
51 NLGRRVLTVK CDVSQPGDVE AFGKQVISTF GRCDILVNNA GIYPLIPFDE
101 LTFEQWKKTF EINVDSGFLM AKAFVPGMKR NGWGRIINLT STTYWLKIEA
151 YTHYISTKAA NIGFTRALAS DLGKDGITVN AIAPSLVRTA TTEASALSAM
201 FDVLPNMLQA IPRLQVPLDL TGAAAFLASD DASFITGQTL AVDGGMVRH

## Patentansprüche

1. Verfahren zur Herstellung von optisch aktiven-Alkanolen der Formel I 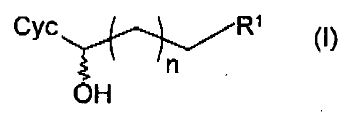 worin
n für einen ganzzahligen Wert von 0 bis 5 steht;
Cyc für einen gegebenenfalls substituierten, ein- oder mehrkernigen, gesättigten oder ungesättigten, carbocyclischen oder heterocyclischen Ring steht, und
R¹ für Halogen, SH, OH, NO₂, NR²R³ oder NR²R³R⁴⁺X⁻ steht, wobei R², R³ und R⁴ unabhängig voneinander für H oder einen Niedrigalkyl- oder Niedrigalkoxy-Rest mit 1 bis 6 kohlenstoffatomen stehen und X⁻ für ein Gegenion steht,
wobei man in einem Alkanon der Formel II 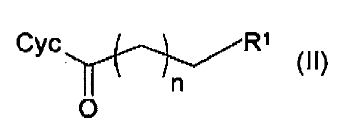 worin n, Cyc und R¹ die oben angegebenen Bedeutungen besitzen,
enthaltenden Medium, ein Enzym (E),mit einer Polypeptidsequenz
(i) SEQ ID NO: 2 oder
(ii) bei der bis zu 25% der Aminosäurereste gegenüber SEQ ID NO:2 durch Deletion, Insertion, Substitution oder einer Kombination davon verändert sind und die noch mindestens 50% der enzymatischen Aktivität von SEQ ID NO:2 besitzt
inkubiert in Gegenwart von Reduktionsäquivalenten, wobei die Verbindung der Formel II zur Verbindung der Formel I enzymatisch reduziert wird, und die im Laufe der Reaktion verbrauchten Reduktionsäquivalente durch Umsetzen eines Opfer-Alkohols zum entsprechenden Opfer-Keton mit Hilfe des Enzyms (E) wieder regeneriert werden und das Opfer-Keton zumindest partiell aus dem Reaktionsmedium entfernt wird, und man das gebildete Produkt (I) isoliert.

2. Verfahren nach Anspruch 1, zur Herstellung von Derivaten des 1-(2-thienyl)-(S)-propanol der Formel III 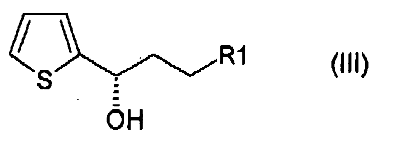 wobei R¹ = Cl oder NHCH₃ bedeuten,
wobei man in einem Derivate des 1-(2-thienyl)-propanon der Formel IV 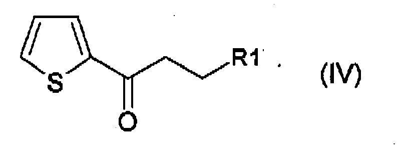 enthaltenden Medium diese Verbindung zur Verbindung der Formel III enzymatisch reduziert wird, und man das in im Wesentlichen enantiomerenreiner Form gebildete Produkt isoliert.

3. Verfahren nach einem der vorhergehenden Ansprüche, wobei das Enzym von einer Nukleinsäuresequenz gemäß SEQ ID NO:1 oder einem funktionalen Äquivalent davon kodiert wird.

4. Verfahren nach einem der vorhergehenden Ansprüche, wobei als Opfer-Alkohol 2-Pentanol eingesetzt wird.

5. Verfahren nach einem der vorhergehenden Ansprüche, wobei man das während der Reaktion aus dem Opfer-Alkohol gebildete Opfer-Keton aus dem Reaktionsmedium destillativ zumindest partiell entfernt.

6. Verfahren nach einem der vorhergehenden Ansprüche, wobei die Reaktion in Gegenwart eines rekombinanten Mikroorganismus durchgeführt wird, der mit einem Nukleinsäurekonstrukt transformiert ist, welches für ein Enzym gemäß der Definition in Anspruch 1 kodiert.

## Claims

1. A process for preparing optically active alkanols of the formula I 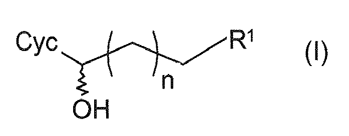 in which
n is an integer from 0 to 5;
Cyc is an optionally substituted, mono- or polynuclear, saturated or unsaturated, carbocyclic or heterocyclic ring, and
R¹ is halogen, SH, OH, NO₂, NR²R³ or NR²R³R⁴⁺X⁻, with R², R³ and R⁴ independently of one another being hydrogen or a low alkyl or low alkoxy radical having from 1 to 6 carbon atoms and X⁻ being a counterion,
which process comprises incubating in a medium comprising alkanone of the formula II 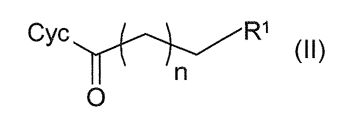 in which n, Cyc and R¹ are as defined above,
an enzyme (E) having a polypeptide sequence
(i) SEQ ID NO: 2 or
(ii) in which, compared to SEQ ID NO:2, up to 25% of the amino acid radicals have been altered by deletion, insertion, substitution or a combination thereof and which retains at least 50% of the enzymic activity of SEQ ID NO:2
in the presence of reduction equivalents, the compound of the formula II being reduced enzymically to give the compound of the formula I and the reduction equivalents consumed in the course of the reaction being regenerated by reacting a sacrificial alcohol to give the corresponding sacrificial ketone with the aid of the enzyme (E) and removing said sacrificial ketone at least partially from the reaction medium, and isolating the product (I) formed.

2. The process according to claim 1, for preparing derivatives of the 1-(2-thienyl)-(S)propanol of the formula III 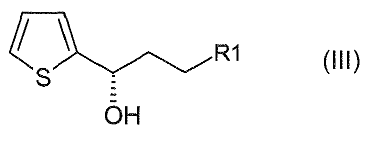 where R¹ = Cl or NHCH₃,
wherein in a medium comprising derivatives of the 1-(2-thienyl)propanone of the formula IV 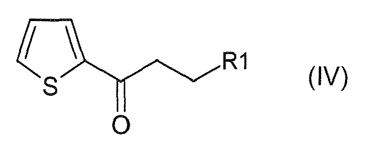 this compound is enzymically reduced to give the compound of the formula III and the essentially enantiomerically pure product formed is isolated.

3. The process according to any of the preceding claims, wherein the enzyme is encoded by a nucleic acid sequence according to SEQ ID NO:1 or by a functional equivalent thereof.

4. The process according to any of the preceding claims, wherein the sacrificial alcohol used is 2-pentanol.

5. The process according to any of the preceding claims, wherein the sacrificial ketone formed from the sacrificial alcohol during the reaction is removed, at least partially, from the reaction medium by distillation.

6. The process according to any of the preceding claims, wherein the reaction is carried out in the presence of a recombinant microorganism which has been transformed with a nucleic acid construct coding for an enzyme according to the definition in claim 1.

## Revendications

1. Procédé pour la préparation d'alcanols optiquement actifs de formule I 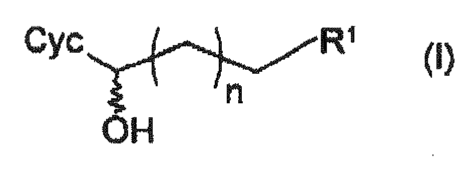 dans laquelle
n représente un nombre entier allant de 0 à 5 ;
Cyc représente un cycle mono- ou polynucléaire, saturé ou insaturé, carbocyclique ou hétérocyclique, éventuellement substitué, et
R¹ représente un atome d'halogène, SH, OH, NO₂, NR²R³ ou NR²R³R⁴⁺X⁻, R², R³ et R⁴ représentant, chacun indépendamment, H ou un radical alkyle inférieur ou alcoxy inférieur ayant de 1 à 6 atomes de carbone et X⁻ représentant un ion opposé,
dans lequel on met à incuber, dans un milieu contenant une alcanone de formule II 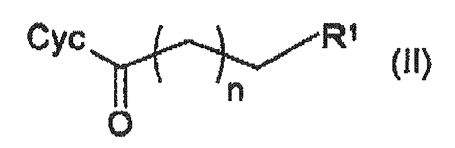 dans laquelle n, Cyc et R¹ ont les significations données plus haut,
une enzyme (E) ayant une séquence polypeptidique
(i) SEQ ID n° 2 ou
(ii) dans laquelle jusqu'à 25 % des résidus aminoacide sont modifiés par rapport à la séquence SEQ ID n° 2 par délétion, insertion, remplacement ou une association de ces modifications et qui possède encore au moins 50 % de l'activité enzymatique de SEQ ID n° 2,
en présence d'équivalents de réduction, de sorte que le composé de formule II est réduit par voie enzymatique en le composé de formule I, et les équivalents de réduction consommés au cours de la réaction sont de nouveau régénérés par conversion d'un alcool sacrificiel en la cétone sacrificielle correspondante à l'aide de l'enzyme (E), et la cétone sacrificielle est au moins partiellement éliminée du milieu réactionnel, et on isole le produit (I) formé.

2. Procédé selon la revendication 1, pour la préparation de dérivés du 1-(2-thiényl)-(S)-propanol de formule III 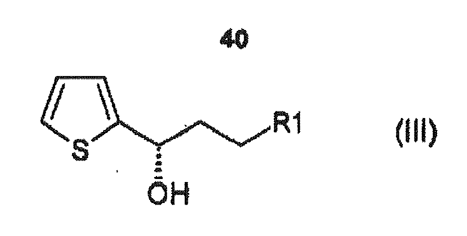 où R¹ représente Cl ou NHCH₃,
dans lequel, dans un milieu contenant des dérivés de la 1-(2-thiényl)-propanone de formule IV 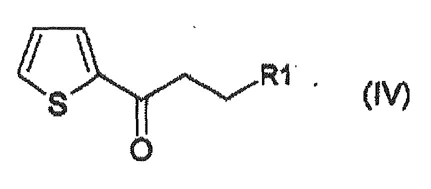 ce composé est réduit par voie enzymatique en le composé de formule III, et on isole le produit formé sous forme essentiellement d'énantiomère pur.

3. Procédé selon l'une quelconque des revendications précédentes, dans lequel l'enzyme est codée par une séquence d'acide nucléique selon SEQ ID n° 1 ou un équivalent fonctionnel de celle-ci.

4. Procédé selon l'une quelconque des revendications précédentes, dans lequel on utilise comme alcool sacrificiel le 2-pentanol.

5. Procédé selon l'une quelconque des revendications précédentes, dans lequel pendant la réaction on élimine au moins partiellement du milieu réactionnel, par distillation, la cétone sacrificielle formée à partir de l'alcool sacrificiel.

6. Procédé selon l'une quelconque des revendications précédentes, dans lequel la réaction est effectuée en présence d'un micro-organisme recombinant qui est transformé par un produit de construction d'acide nucléique qui code pour une enzyme selon la définition donnée dans la revendication 1.
